# EUROPÄISCHE PATENTSCHRIFT

(11) **EP 3 919 105 B1**
(45) Veröffentlichungstag und Bekanntmachung des Hinweises auf die Patenterteilung: **13.03.2024**
(21) Anmeldenummer: 21000138.4
(22) Anmeldetag: 17.05.2021
(51) Int. Cl.: A61M 16/00

(54) **SCHNITTSTELLE FÜR BEATMUNGSGERÄT**
INTERFACE FOR VENTILATOR
INTERFACE POUR UN APPAREIL RESPIRATOIRE

(30) Priorität: 06.06.2020 DE 102020003438
(43) Veröffentlichungstag der Anmeldung: 08.12.2021
(73) Patentinhaber: Löwenstein Medical Technology S.A., 2557 Luxembourg (LU)
(72) Erfinder: Adametz, Benjamin, 22609 Hamburg (DE); Alberts, John, 21465 Reinbek (DE)
(74) Vertreter: Marx, Thomas

(56) Entgegenhaltungen:
- WO-A1-2015/167388
- WO-A1-2020/082317
- WO-A2-2007/038152
- US-A1- 2002 144 682
- US-A1- 2019 307 981

## Beschreibung

Die Erfindung betrifft eine Schnittstelle für Beatmungsgeräte. In der vorliegenden Erfindung wird unter einer Schnittstelle ein Datenübergabepunkt verstanden, die es dem medizinischen Fachpersonal ermöglicht, sowohl Patientendaten zu empfangen als auch aktiv sowie teilautomatisiert grundlegende Funktionen der medizinischen Geräte patientenfern zu steuern und zu überwachen.

Nachfolgend ist unter Beatmungsgerät jedwedes Gerät zu verstehen, welches einen Anwender bzw. Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiPAP-Geräte, Narkose- bzw. Anästhesiegeräte, Atemtherapiegeräte, (klinische, außerklinische oder Notfall) Beatmungsgeräte, Highflow-Therapiegeräte und Hustenmaschinen.

Ein bekanntes Beatmungsgerät wird in WO 2007/038152 A2 vorgestellt.

Bei den bisher bekannten Beatmungsgeräten wird zur Datenfernübertragung ein in das Gerät implementiertes Modem oder auf den elektronischen Boards fest verbaute Wifi/WLAN Module genutzt. Der Nachteil einer solchen Lösung ist, dass Modems bei einem Defekt schwer zu tauschen sind und in aller Regel, den zum Zeitpunkt der Anschaffung des Beatmungsgeräts technischen Standard abbilden. Hinzu kommt, dass in verschiedenen Staaten auch unterschiedliche Netz- und Zulassungsstandards verbreitet sind, das fest verbaute Modem oder Modul also jeweils entsprechend angepasst sein müsste. Hinzu kommt, dass Beatmungsgeräte in den allermeisten Fällen voll konfiguriert sind d.h. alle verfügbaren Funktionen sind in einem solchen medizinischen Gerät verbaut. Dies gilt sowohl für werksseitige Softwareapplikationen als auch für Hardwarekomponenten und somit auch für das bereits genannte Modem. Da es sich bei Beatmungsgeräten um Investitionsgüter handelt, die über einen langen Zeitraum genutzt werden, ist es geboten eine Möglichkeit zu schaffen, den Datenaustausch möglichst nah am Stand der Technik zu halten. Dies gilt eben nicht nur für die Aktualisierung der Gerätesoftware, sondern auch für das meist von Zulieferern hinzugekaufte Modem. Einmal verbaut, bleibt es über den gesamten Nutzungszeitraum im Beatmungsgerät verbaut. Ein Austausch gegen eine aktuelle Modemversion ist aufwendig und teuer und unterbleibt daher häufig. Hierbei kann es sogar passieren, dass Modems so veraltet sind das sie überhaupt nicht mehr nutzbar sind und diese Funktion somit nicht mehr an dem Beatmungsgerät zur Verfügung steht. Sollte diese Funktion jedoch benötigt werden ist es aufwendig zunächst einmal zu prüfen ob ein mit dieser Funktionalität ausgestattetes Beatmungsgerät zur Verfügung steht. Im ungünstigsten Fall ist ein Wechsel unter laufender Beatmung notwendig um dem medizinischen Fachpersonal im Bedarfsfall die Möglichkeit zu geben ein entsprechend ausgestattetes Gerät heranzuführen.

Die Verwendung eines Modems dient dazu, wenn es notwendig oder angeraten ist, eine Steuerung und Überwachung des Patienten patientenfern zu betreiben. Mit patientenfern ist hierbei insbesondere die Bedienung eines Beatmungsgeräts über eine größere Distanz gemeint und bei dem nicht zwingend die Nähe zum medizinischen Fachpersonal vorhanden sein muss.

Die Verwendung einer Datenübertragungsschnittstelle hat somit den Vorteil, dass diese nur bei Bedarf mit einem dann jederzeit zu beschaffenden Datenübertragungsstick ausgestattet werden kann und die Datenübertragung stets nach dem neuesten Stand der Technik erfolgt. Die Nutzung eines Datenübertragungsstick hilft somit auch Ressourcen einzusparen.

Die Aufgabe der Erfindung besteht somit darin, eine von außen leicht zugängliche, standardisierte Schnittstelle für Datenübertragungssticks zu schaffen.

Diese Aufgabe wird durch den Gegenstand mit den Merkmalen des Anspruchs 1 gelöst.

Beansprucht wird ein Beatmungsgerät mit einem Gehäuse, wenigstens einer Atemgasquelle, wenigstens einem Steuersystem, wenigstens einer Schnittstelle, wobei das Gehäuse eine Aufnahme für die Schnittstelle aufweist und die Aufnahme mit der Schnittstelle in einer Vertiefung des Gehäuses angeordnet ist. Die Vertiefung ist als Schacht ausgebildet und der Schacht weist eine Öffnung auf, wobei sich von der Öffnung in Richtung eines Innenraumes des Beatmungsgerätes zumindest zwei Umfangwände bis zu einem Boden erstrecken und wobei die Aufnahme mit der Schnittstelle im Bereich des Bodens oder einer Umfangswand der Vertiefung angeordnet ist. Als Schacht wird dabei ganz allgemein ein Raum angesehen, welcher von einer Öffnung aus zu den Seiten durch mindestens zwei Wände zumindest teilweise umgeben ist und einen der Öffnung gegenüberliegenden Boden aufweist. Ein solcher Schacht kann so beispielsweise über nur eine offene Seite verfügen, aber auch beispielsweise drei oder mehr offene Seiten aufweisen.

In einer bevorzugten Ausführungsform des Beatmungsgeräts weist zumindest eine Umfangswand Führungsmittel auf, um einen Datenübertragungsstick und/oder einen Datenspeicherstick zu führen und im montierten Zustand zu stützen.

In einer bevorzugten Ausführungsform des Beatmungsgeräts ist die Vertiefung durch einen Deckel verschlossen.

In manchen Ausführungsformen des Beatmungsgeräts ist die Vertiefung durch einen mit dem Gehäuse im Wesentlichen flächenbündig abschließenden Deckel verschlossen. Soweit nicht ausdrücklich anders angegeben bedeutet flächenbündig abschließend im Wesentlichen, dass zwischen der Fläche, welche die Außenseite des Geräts darstellt und der Fläche, welche durch die Außenseite des Deckels dargestellt wird, kein bzw. kein größerer Absatz/Höhenunterschied zu finden ist. Ein noch als flächenbündig anzusehender Absatz/Höhenunterschied kann beispielsweise im Bereich von 2 mm sein. Davon ausgenommen sind etwaige Armaturen oder Strukturierungen der Oberfläche des Deckels, zum Beispiel um ein Öffnen des Deckels zu erleichtern, sowie eine Strukturierung der Außenfläche des Gehäuses, zum Beispiel durch eine gewisse Rauigkeit. Auch ist unter dem flächenbündigen Abschluss des Deckels zu verstehen, dass die Kanten des geschlossenen Deckels nah an den Kanten des Gehäuses liegen. Beispielsweise ist zwischen den Kanten des Deckels und den Kanten des Gehäuses ein maximaler Abstand von 2 mm. Zum Beispiel kann dieser Abstand auch in Form einer Nut ausgeprägt sein.

In manchen Ausführungsformen des Beatmungsgeräts ist der Deckel relativ zum Gehäuse bewegbar angeordnet und an dem Gehäuse befestigt.

In manchen Ausführungsformen des Beatmungsgeräts verfügt der Deckel über zumindest eine Armatur oder eine Oberflächenstrukturierung, um ein Öffnen des Deckels zu vereinfachen. Eine Armatur kann zum Beispiel eine schmale Erhöhung sein, welche mit den Fingern zu greifen ist. Eine Oberflächenstrukturierung kann beispielsweise durch eine raue Oberfläche erzielt werden, welche einen gewissen Gleitwiderstand zwischen Finger und Deckeloberfläche erzeugt. Auch ein Anbringen von zum Beispiel Rillen auf der Oberfläche des Deckels kann eine solche Oberflächenstrukturierung sein.

In manchen Ausführungsformen des Beatmungsgeräts ist im Gehäuse und/oder der Umfangswand ein Mechanismus eingebaut, welcher bei Druckausübung den Deckel zumindest teilweise öffnet und bei erneuter Druckausübung wieder in die Ausgangsposition zurückkehrt. So kann beispielsweise ein Deckel angebracht werden, welcher beispielsweise flächenbündig mit dem Gehäuse abschließt und ohne extra Armatur oder ähnliches auskommt aber dennoch leicht zu öffnen ist. Beispielsweise wäre eine Art Federdruckelement zu verwenden, welches beim Drücken zum Öffnen des Deckels eine Art Bolzen durch eine Feder bewegt, welcher wiederum den Deckel zumindest leicht anhebt, sodass dieser dann zugreifen wäre. Beim Schließen des Deckels kann durch Drücken der Bolzen sowie die Feder wieder in die ursprüngliche Position zurückgeführt werden.

In manchen Ausführungsformen des Beatmungsgeräts ist der Deckel mit einer umlaufenden Dichtung versehen.

In einer bevorzugten Ausführungsform des Beatmungsgeräts weist die Schnittstelle einen Spritzwasser- und Berührungsschutz auf. Durch den Spritzwasser- und Berührungsschutz soll das unbeabsichtigte Eindringen von Wasser sowie Fremdkörpern in das Innere des Beatmungsgeräts verhindert werden. Auch ein Hineingreifen in das Innere des Beatmungsgerätes durch die Schnittstelle wäre so zu verhindern. In manchen Ausführungsformen kann der Spritzwasser- und Berührungsschutz auch durch eine entsprechend ausgebildete Vertiefung und/oder Deckel, beispielsweise mit Dichtung, erreicht werden. Auch eine entsprechende Abdichtung bei der Schnittstelle bzw. im Bereich der Aufnahme ist als Spritzwasser- und Berührungsschutz möglich. In weiteren Ausführungsformen kann der Berührungsschütz die Klasse IP1X oder höher erreichen und/oder der Spritzwasserschutz die Klasse IPX1 oder höher erreichen.

In manchen Ausführungsformen des Beatmungsgeräts ist die Aufnahme mit der Schnittstelle sowie die Vertiefung gegenüber dem Innenraum des Beatmungsgeräts abgedichtet.

In manchen Ausführungsformen des Beatmungsgeräts ist der Deckel mit einem Werkzeug verschließbar. Beispielsweise könnte der Deckel mit einer Schraube verschließbar sein, welche in eine entsprechend dafür vorgesehene Position durch den Deckel in das Gehäuse eingeschraubt wird.

In manchen Ausführungsformen des Beatmungsgeräts ist der Deckel durch ein Schloss verschließbar.

In manchen Ausführungsformen des Beatmungsgeräts ist der Deckel als Gleitschieber ausgebildet.

In manchen Ausführungsformen des Beatmungsgeräts rastet der Deckel im geschlossenen Zustand in einer entsprechenden Vorrichtung in den Umfangswänden und/oder dem Gehäuserand und/oder im/am Gehäuse ein.

In manchen Ausführungsformen des Beatmungsgeräts ist der Deckel durch ein Scharnier mit dem Gehäuse verbunden.

In manchen Ausführungsformen des Beatmungsgeräts ist die Vertiefung in der Außenfläche des Gehäuses beabstandet zum Gehäuserand angeordnet. So ist beispielsweise der Schacht an allen vier Seiten von einer Umfangswand umgeben und nur im Bereich der Öffnung offen. Das heißt beispielsweise, dass ein Einführen bzw. Entnehmen eines Datenübertragungssticks oder eines Datenspeichersticks nur durch die Öffnung möglich ist.

In manchen Ausführungsformen des Beatmungsgeräts ist die Vertiefung Teil des Gehäuserandes. Das heißt beispielsweise, dass, soweit kein Deckel vorhanden ist, die Vertiefung zu zumindest zwei Seiten - der Öffnung und dem Teil, welcher Teil des Gehäuserandes ist - offen ist.

In manchen Ausführungsformen des Beatmungsgeräts dehnt sich die Vertiefung zumindest teilweise über zwei Längsseiten des Gehäuserandes aus. Beispielsweise wäre es der Fall, wenn die Vertiefung an einer Ecke des Gehäuses angeordnet ist und an dieser Stelle zwei der Umfangswände nicht vorhanden sind.

In manchen Ausführungsformen des Beatmungsgeräts ermöglicht die Schnittstelle eine Kontaktierung mit einer Vielzahl unterschiedlicher Datenübertragungssticks.

In manchen Ausführungsformen des Beatmungsgeräts ermöglicht die Schnittstelle eine Kontaktierung, mit einer Vielzahl unterschiedlicher Datenspeichersticks, beispielsweise USB-Sticks.

In manchen Ausführungsformen des Beatmungsgeräts ist die Aufnahme derart in dem Gehäuse versenkt angeordnet, dass ein Datenübertragungsstick der in der Aufnahme und in der Schnittstelle steckt, vollständig in der Vertiefung angeordnet ist und maximal flächenbündig mit der Gehäuseoberfläche des Gehäuses abschließt, nicht aber über die Gehäuseoberfläche hinausragt.

In manchen Ausführungsformen des Beatmungsgeräts verfügt die Schnittstelle über Eingriffsbereiche für Finger und/oder Daumen für eine einfache Zug-Entnahme des Datenübertragungssticks und/oder Datenspeichersticks.

In manchen Ausführungsformen des Beatmungsgeräts verfügt die Schnittstelle und/oder die Aufnahme und/oder die Vertiefung über eine Entnahmevorrichtung mit Rückstellfunktion.

In manchen Ausführungsformen des Beatmungsgeräts wird der Datenübertragungsstick durch die Betätigung der Entnahmevorrichtung in eine Position geführt, in der dieser nicht mehr flächenbündig mit dem Gehäuse abschließt oder innerhalb des Gehäuses angeordnet ist, sondern zumindest teilweise über das Gehäuse hinausragt.

In manchen Ausführungsformen des Beatmungsgeräts verbindet die Schnittstelle den Datenübertragungsstick mechanisch und elektrisch.

In manchen Ausführungsformen des Beatmungsgeräts verbindet die Schnittstelle den Datenübertragungsstick elektrisch und optional mechanisch mit einer im Beatmungsgerät vorhandenen elektronischen Leiterplatte direkt oder über eine Kabelverbindung.

In manchen Ausführungsformen des Beatmungsgeräts weist die Schnittstelle einen Dauerstromkontakt und/oder einen Automatikstromkontakt auf, wobei dem Automatikstromkontakt ein Sensor zugeordnet ist, der zum Erfassen einer mechanischen Belegung der Schnittstelle ausgebildet ist.

In manchen Ausführungsformen des Beatmungsgeräts steht die Schnittstelle mit einer Steuereinheit des Beatmungsgerätes elektrisch leitend in Kontakt und ist zur Kommunikation von Datensignalen mit dem Beatmungsgerät ausgebildet.

In manchen Ausführungsformen des Beatmungsgeräts ist der Datenübertragungsstick als ein in die Aufnahme einführbares und in die Schnittstelle steckbares Einführmodul ausgeführt.

In manchen Ausführungsformen des Beatmungsgeräts stellt die Schnittstelle durch Verbindung eines Datenübertragungssticks eine Sende-/Empfangsanlage dar, die entweder mit einem Ethernet- und/oder Funk- und/oder GSM- und /oder UMTS- und/oder 4G- und/oder 5G-Netz verbunden werden kann.

In manchen Ausführungsformen des Beatmungsgeräts ist der Zugriff auf die Daten eines in der Schnittstelle befindlichen Datenübertragungsstick durch Passwort und/oder Hardware-Verschlüsselung geschützt.

In manchen Ausführungsformen des Beatmungsgeräts kann die Schnittstelle sowohl für einen Datenübertragungsstick als auch für einen Datenspeicher verwendet werden, auf dem Patientendaten temporär gespeichert werden können, um diese über andere Kanäle weiter zu verteilen.

In manchen Ausführungsformen des Beatmungsgeräts weist die Schnittstelle und/oder die Aufnahme und/oder der Boden und/oder die Vertiefung und/oder der Schacht und/oder zumindest eine Umfangswand Mittel auf und/oder ist eingerichtet, gegen elektrische Felder sowie hochfrequente Wellen abzuschirmen.

In manchen Ausführungsformen des Beatmungsgeräts ist die Schnittstelle (6) und/oder die Aufnahme und/oder der Boden und/oder die Vertiefung und/oder der Schacht und/oder zumindest eine Umfangswand ausgebildet, elektrische Felder oder Wellen nur in Richtung der Öffnung zu ermöglichen.

In manchen Ausführungsformen des Beatmungsgeräts kann die Schnittstelle auch dazu genutzt werden die Geräte Software mittels Memory Stick von einem Gerät auf ein anderes Gerät zu übertragen.

In manchen Ausführungsformen des Beatmungsgeräts kann der Deckel elektronisch verriegelt werden.

In manchen Ausführungsformen des Beatmungsgeräts kann die elektronische Verriegelung des Deckels durch eine Authentifizierung aufgehoben werden.

In manchen Ausführungsformen des Beatmungsgeräts wird die elektronische Verriegelung des Deckels nur aktiviert, wenn ein Datenübertragungsstick und/oder ein Datenspeicherstick mit der Schnittstelle verbunden ist.

In manchen Ausführungsformen des Beatmungsgeräts ist die Aufnahme im Deckel angeordnet, wobei der Deckel mit dem Gehäuse verbunden ist.

In manchen Ausführungsformen des Beatmungsgeräts dehnt sich der Deckel zumindest über zwei Längsseiten des Gehäuserandes aus.

In manchen Ausführungsformen des Beatmungsgeräts ist die Aufnahme beweglich in der Vertiefung angeordnet.

In manchen Ausführungsformen des Beatmungsgeräts bewegt sich die Aufnahme beim Öffnen des Deckels in Richtung Gehäuseoberfläche des Gehäuses.

In manchen Ausführungsformen des Beatmungsgeräts ist die Aufnahme an einem Kabel angeordnet, welches elektrisch mit dem Beatmungsgerät bzw. entsprechenden Steuereinheiten verbunden ist und zumindest teilweise in der Vertiefung angeordnet ist, sodass die Aufnahme zumindest teilweise bis außerhalb der Vertiefung gezogen werden kann.

In manchen Ausführungsformen des Beatmungsgeräts ist in der Vertiefung zusätzlich zu der Schnittstelle zumindest ein weiteres Bauteil und/oder ein weiterer Anschluss angeordnet ist.

In manchen Ausführungsformen des Beatmungsgeräts ist der Bereich der Schnittstelle in der Vertiefung zumindest teilweise durch eine zumindest teilweise ausgebildete Zwischenwand von den weiteren Bauteilen und/oder Anschlüssen räumlich getrennt.

In manchen Ausführungsformen des Beatmungsgeräts wird die Vertiefung teilweise von der Gehäuseoberfläche verdeckt.

In manchen Ausführungsformen des Beatmungsgeräts ist die Aufnahme mit der Schnittstelle in bzw. an einer der Umfangswände angeordnet, wobei die Vertiefung in dem Bereich der Umfangswand, in bzw. an welcher die Aufnahme mit der Schnittstelle angeordnet ist, von der Gehäuseoberfläche verdeckt wird.

In manchen Ausführungsformen ist in der Vertiefung und/oder am Deckel zumindest ein Schaumstoffelement angeordnet. Ein solches Schaumstoffelement kann beispielsweise dazu dienen, den in die Schnittstelle eingesteckten Datenübertragungsstick bzw. dem Datenspeicherstick zu haltern bzw. gegenüber Bewegungen, welche zu einem unbeabsichtigten Lösen der Verbindung zwischen Datenübertragungsstick und Datenspeicherstick führen können, zu fixieren.

In manchen Ausführungsformen des Beatmungsgeräts ist am Deckel und/oder in der Vertiefung zumindest ein Federelement zur Fixierung des Datenübertragungssticks bzw. des Datenspeichersticks angebracht.

Der Gegenstand der vorliegenden Erfindung ist also eine Schnittstelle an dem Beatmungsgerät, die durch Ihre Form und Ausprägung einerseits und der Möglichkeit eine Vielzahl unterschiedlicher den allgemeinen Standards entsprechende Datenübertragungssticks zu nutzen andererseits in der Lage ist, Daten zu empfangen und zu versenden. Bei der Auswahl der genauen Lage am Beatmungsgerät ist insbesondere auf eine gute Zugänglichkeit zu achten.

Es kann somit auf ein von außen nicht zugängliches Modem verzichtet werden.

Da die mit der erfindungsgemäßen Schnittstelle ausgestatteten Beatmungsgeräte in aller Regel in einem intensivmedizinischen Bereich und in einem Homecare-Umfeld betrieben werden, sind Fehlbedienungen und Störungen im Zusammenhang mit der Nutzung der Schnittstelle soweit möglich auszuschließen. Mit Fehlbedienungen sind in diesem Zusammenhang Störungen gemeint, die in einem Bedienfehler des medizinischen Fachpersonals begründet sind. Als Störungen sind grundsätzlich alle weiteren Fehler gemeint die nicht auf einen Bedienfehler zurückzuführen sind und durch äußere Einflüsse entstehen.

Die Schnittstelle sollte daher den erhöhten Ansprüchen an Sicherheit im medizinischen Gerätebau entsprechen, ohne hierdurch Abstriche an Bedienkomfort und Funktionalität machen zu müssen.

Die Schnittstelle sollte somit, an einer für das Bedienpersonal bzw. technisches Personal gut zugänglichen Stelle des Beatmungsgeräts angeordnet sein und eine leicht zu bedienende Steckmöglichkeit bieten. Die Schnittstelle könnte demnach in einem Ausführungsbeispiel sowohl in der Gehäusefläche von vier Umfangswänden begrenzt liegen aber auch, in dem den Gehäuseflächenrand bildenden Bereich angesiedelt sein und damit nur von drei Umfangswänden begrenzt werden. Ebenfalls denkbar wäre es, die Schnittstelle in einem Eckbereich des Gehäuses zu platzieren. Folglich würden hier nur zwei Umfangswände eine seitliche Begrenzung bilden.

Der mechanische Zugriff auf den Datenübertragungsstick sollte ungeachtet der genauen Ausprägung d.h. Länge, Breite, Höhe, Größe des Datenübertragungssticks für Berechtigte problemlos möglich sein. Er steckt außerdem so im Gehäuse, dass er vorteilhafterweise nicht über den Gehäuserand herausragt um zu verhindern, dass er versehentlich durch mechanische Kräfte beschädigt oder entfernt wird. Es ist zudem in einer weiteren vorteilhaften Ausführungsform möglich, den Stick gegen unautorisiertes entfernen und Manipulation von außen zu sichern.

Grundsätzlich sollte die Entnahme des Datenübertragungssticks regelmäßig ohne Werkzeug für Berechtigte möglich sein. In manchen Ausführungsformen kann aber auch eine Entnahme nur durch Einsatz von Werkzeug möglich sein.

Denkbar wäre in diesem Zusammenhang auch ein Zurückhalten/Sperren des Datenübertragungssticks ähnlich der bei Automatikfahrzeugen benutzten Funktionalität der verhinderten Freigabe des Schlüssels, solange der Wählhebel sich nicht im Parkmodus befindet. Eine Freigabe des Datenübertragungssticks respektive des Datenspeichersticks zur Entnahme ist somit erst möglich, wenn sich das medizinische Fachpersonal authentifiziert hat und somit berechtigt ist den Stick aus der Schnittstelle zu entnehmen.

Neben der vorgenannten elektromechanischen Sicherung des Sticks sind jedoch auch rein mechanische Sicherungen wie ein verschließen des Schachtes mit einem Deckel in unterschiedlichen Ausführungsformen möglich.

Um den Schacht und insbesondere die Kontaktierungen vor Feuchtigkeit zu schützen ist der Deckel mit einer umlaufenden Gummidichtung ausgestattet. Dabei muss die umlaufende Gummidichtlippe nicht zwingend deckelseitig angebracht sein.

Erreicht wird dies durch eine in einer Vertiefung sitzenden Schnittstelle in unterschiedlicher Ausprägung und Ausdehnung.

Ziel aller vorgenannten Vorgehensweisen ist es, die Sicherheit bei der Nutzung der Funktionalitäten des Datenübertragungssticks sicherzustellen, ohne die Handhabung unnötig zu erschweren.

Erreicht wird dies weiter durch eine Abdeckung die unverlierbar mit dem Gehäuse des medizinischen Gerätes verbunden ist und die einerseits die unautorisierte Entnahme des Sticks als auch durch in die Abdeckung bzw. in das Gehäuse integrierte umlaufende Dichtlippen, einen Schutz gegen eindringendes Wasser bzw. Feuchtigkeit bietet.

Nachfolgend wird die in einem Beatmungsgerät vorgeschlagenen Schnittstelle anhand verschiedener, teilweise stark vereinfachter, Zeichnungen beispielhaft näher erläutert. Hierin zeigt:
- Fig. 1: Eine beispielhafte Ausführungsform des beanspruchten Beatmungsgeräts in einer vereinfachten perspektivischen Ansicht mit dem Gehäuse 1 und der als Schacht 10 ausgeführten Vertiefung 9.
- Fig. 2: Draufsicht auf das Beatmungsgerät aus Fig. 1 mit der Schnittstelle 6 in der Aufnahme 7.
- Fig. 3: Querschnitt A-A durch das Gehäuse 1 aus Fig. 2.
- Fig. 4: Draufsicht auf eine beispielhafte Ausführungsform des Beatmungsgeräts mit Datenübertragungsstick 19 bzw. Datenspeicherstick 20.
- Fig. 5: Querschnitt A-A durch das Gehäuse 1 aus Fig. 4 mit Deckel 16.
- Fig. 6: Querschnitt A-A durch das Gehäuse 1 aus Fig. 4 mit Deckel 16 und umlaufender Dichtung 17.
- Fig. 7: Draufsicht auf eine beispielhafte Ausführungsform des Beatmungsgeräts mit der Vertiefung 9 als Teil der Außenfläche 30 im Gehäuserand 18 des Gehäuses 1.
- Fig. 8: Querschnitt A-A durch das Gehäuse 1 aus Fig. 7 mit Datenübertragungsstick 19 bzw. Datenspeicherstick 20.
- Fig. 9: Draufsicht auf eine beispielhafte Ausführungsform des Beatmungsgeräts mit der Vertiefung 9 als Teil von zwei Außenflächen 30 im Gehäuserand 18.
- Fig. 10: Querschnitt A-A durch das Gehäuse 1 aus Fig. 9.
- Fig. 11: Perspektivische Ansicht einer beispielhaften Ausführungsform des Beatmungsgeräts mit der Vertiefung 9 als Teil von zwei Außenflächen 30 des Gehäuserands 18.
- Fig. 12: Querschnitt durch eine beispielhafte Ausführungsform des Beatmungsgeräts mit Gehäuse 1 und Eingriffsbereichen 22 an der Vertiefung 9.
- Fig. 13: Querschnitt durch eine nicht von der beanspruchten Erfindung erfasste beispielhafte Ausführungsform des Beatmungsgeräts mit einer im Deckel 16 angeordneten Aufnahme 7 mit Schnittstelle 6.
- Fig. 14: Querschnitt durch eine beispielhafte Ausführungsform des Beatmungsgeräts mit einer an einem Kabel 25 angeordneten Aufnahme 7 mit Schnittstelle 6.
- Fig. 15: Aufsicht auf eine beispielhafte Ausführungsform des Beatmungsgeräts mit einem als Gleitschieber ausgebildeten Deckel 16.
- Fig. 16: Querschnitt A-A durch das Gehäuse aus Fig. 15 mit als Gleitschieber ausgebildetem Deckel 16, welcher teilweise geschlossen ist.
- Fig. 17: Querschnitt durch eine beispielhafte Ausführungsform des Beatmungsgeräts mit der Vertiefung 9 als Teil der Außenfläche 30 im Gehäuserand 18 und dem Deckel 16 ausgebildet als Gleitschieber.

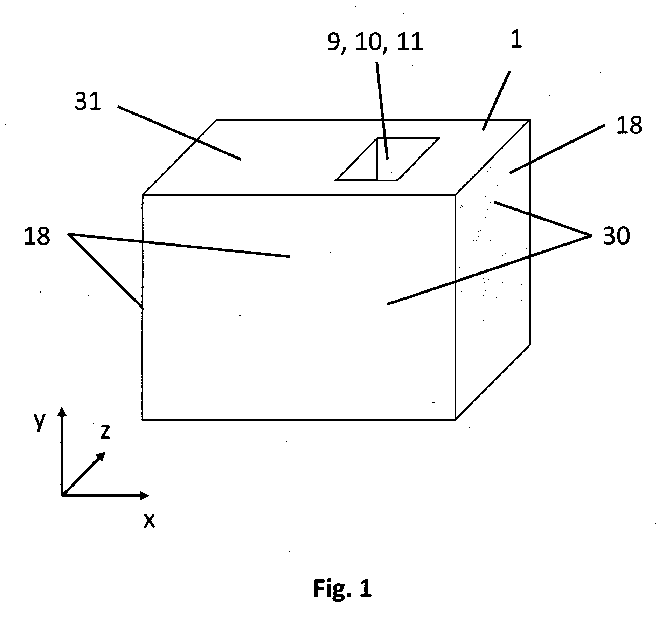

Die Figur 1 zeigt eine stark vereinfachte schematische Darstellung einer beispielhaften Ausführungsform des Beatmungsgeräts bzw. des Gehäuses 1 in einer perspektivischen Darstellung. Die beispielsweise als Schacht 10 ausgeführte Vertiefung 9, in welcher die Schnittstelle 6 angeordnet ist, ist dabei in der als Gehäuseoberfläche 31 bezeichneten Seite des Gehäuses 1 angeordnet. Angrenzend an diese Gehäuseoberfläche 31 verläuft der Gehäuserand 18, welcher gleichzeitig auch die Außenflächen 30 darstellt. Die Anordnung der Vertiefung 9 an der mit Gehäuseoberfläche 31 bezeichneten Oberseite des Gehäuses ist hier nur beispielhaft zu verstehen. Genauso kann die Vertiefung 9 auch an einer der Außenseiten oder an der Unterseite des Gehäuses 1 angeordnet sein. Zur Gehäuseoberfläche 31 hin weist die Vertiefung eine Öffnung 11 auf, durch welche ein Datenübertragungsstick 19 oder ein Datenspeicherstick 20 in die Vertiefung eingebracht werden kann. Ein Datenübertragungsstick 19 kann dabei beispielsweise ein WLAN-Stick sein, welcher eine Verbindung zu Drahtlosnetzwerken ermöglicht oder auch ein sogenannter Surfstick, welcher beispielsweise eine Verbindung zu 4G/5G-Netzwerken ermöglichen kann. Auch andere Ausführungen des Datenübertragungssticks 19, wie zum Beispiel für Bluetooth- oder LPWAN- oder andere Drahtlosverbindungen, sind hier denkbar und möglich. Dazu verfügt die Vertiefung 9 Führungsmittel, welche den Datenübertragungsstick 19 in die Aufnahme 7 bzw. die Schnittstelle 6 führen. Diese Führungsmittel sind so eingerichtet, dass der Datenübertragungsstick 19 oder der Datenspeicherstick 20 im montierten Zustand stützen oder haltern oder beim Haltern unterstützen. Beispielsweise sollte die Vertiefung 9 mit der Schnittstelle 6 bzw. die Öffnung 11 auch im Betrieb des Beatmungsgeräts einigermaßen leicht zugänglich angeordnet sein. Die stark vereinfachte Darstellung des beispielhaften Gehäuses 1 als Quader dient lediglich Ansichtszwecken und schließt andere mögliche Formen nicht aus. Das Gehäuse 1 kann darüber hinaus auch jede andere denkbare Form annehmen.

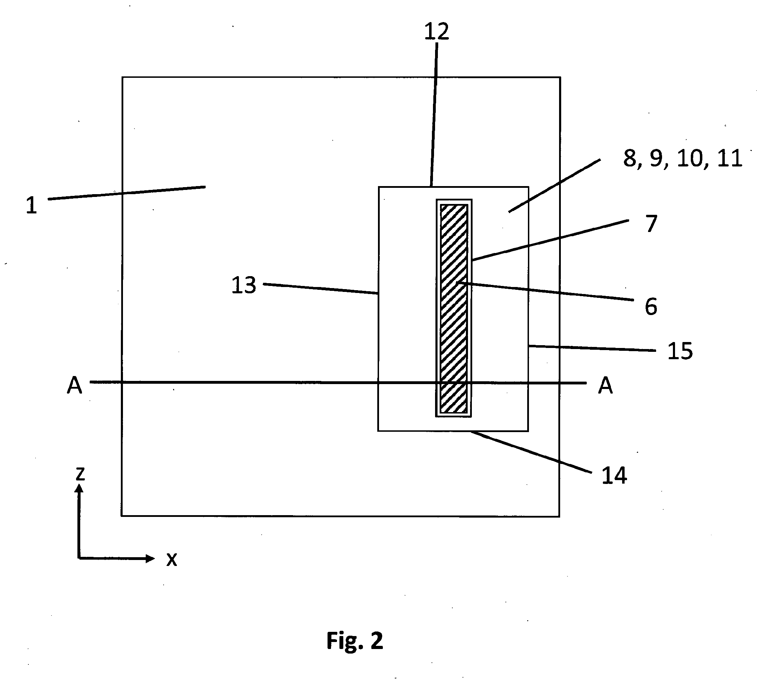

Eine Draufsicht des beispielhaften Gehäuses 1 ist in Figur 2 gezeigt. Die als Schacht 10 ausgeführte Vertiefung 9 ist beabstandet von dem Gehäuserand 19 angeordnet und wird dabei zu den Seiten von Umfangswänden 12, 13, 14, 15 begrenzt, welche sich von der Öffnung 11 in Richtung Innenraum des Beatmungsgeräts bis zu dem Boden 8 erstrecken. Im Bereich des Bodens 8 ist eine Aufnahme 7 mit der Schnittstelle 6 angeordnet. Die Schnittstelle 6 ist beispielsweise so eingerichtet, dass eine Vielzahl von möglichen Datenübertragungssticks 19 und Datenspeichersticks 20 mit der Schnittstelle verbunden werden kann. Beispielsweise verfügt die Schnittstelle dabei über einen USB-Anschluss und/oder einen Lightning-Anschluss. Die Schnittstelle 6 ermöglicht eine Kommunikation von Datensignalen zwischen dem Beatmungsgerät und dem Datenübertragungsstick 19 bzw. dem Datenspeicherstick 20. Neben der elektrischen Verbindung stellt die Schnittstelle 6 beispielsweise auch zusätzliche eine Möglichkeit der mechanischen Verbindung mit einem Datenübertragungsstick 19 oder einem Datenspeicherstick 20 dar. Die Aufnahme 7 kann dabei in verschiedenen Varianten ausgeführt sein. Beispielsweise, andere Ausführungsformen aber nicht ausschließend, kann es sich um einen Durchbruch im Boden 8 zum Innenraum des Beatmungsgeräts handeln, welcher die Schnittstelle 6 umgibt. Zwischen der Aufnahme 7 und der eigentlichen Schnittstelle 6 können zum Beispiel auch Dichtungen angebracht sein, sodass ein Berührungs- und Spritzwasserschutz gewährleistet sein kann. Neben einem Durchbruch im Boden 8, ist es beispielsweise auch möglich, dass die Aufnahme 7 samt Schnittstelle 6 leicht über den Boden 8 überstehen und in den durch die Vertiefung 9 definierten Raum hineinragen. Zudem ist die Aufnahme 7 so ausgebildet, sein, dass sie die Halterung eines eingesteckten

Datenübertragungssticks 19 oder Datenspeicherstick 20 zusätzlich unterstützt oder auch vollständig übernimmt. Die Vertiefung 9 ist beispielhaft mit einem rechteckigen Querschnittsprofil gezeigt. Darüber hinaus kann das Querschnittsprofil aber auch ovale oder runde Formen annehmen oder auch über eine polyedrische Form oder eine Freiform verfügen. Das Querschnittsprofil kann auch von der Öffnung 11 ausgehend Richtung Boden 8 gehend variabel sein. Beispielsweise kann die Querschnittsfläche im Bereich der Öffnung 11 größer sein und sich in Richtung Boden 8 verringern. Dies ist zum Beispiel der Fall, wenn die Vertiefung über zusätzliche Eingriffsbereiche 22 verfügt.

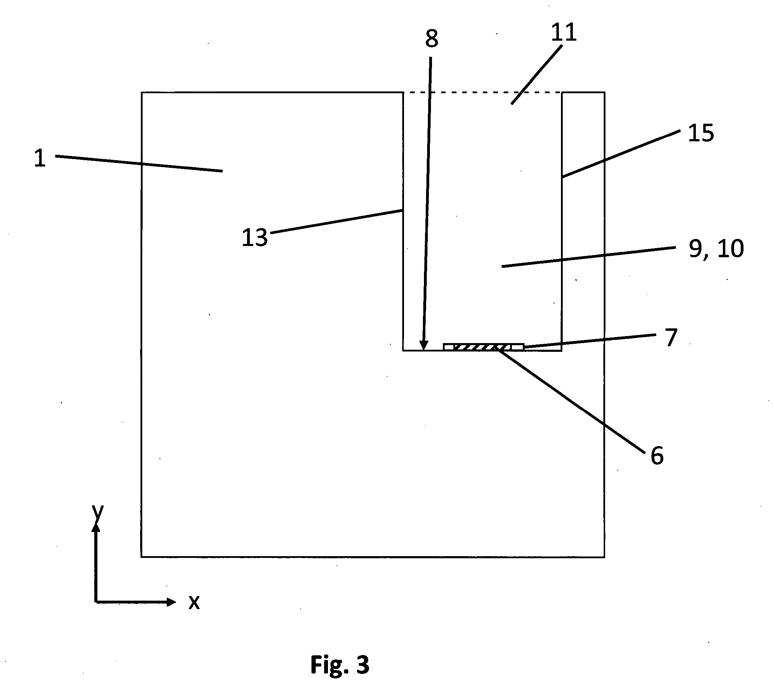

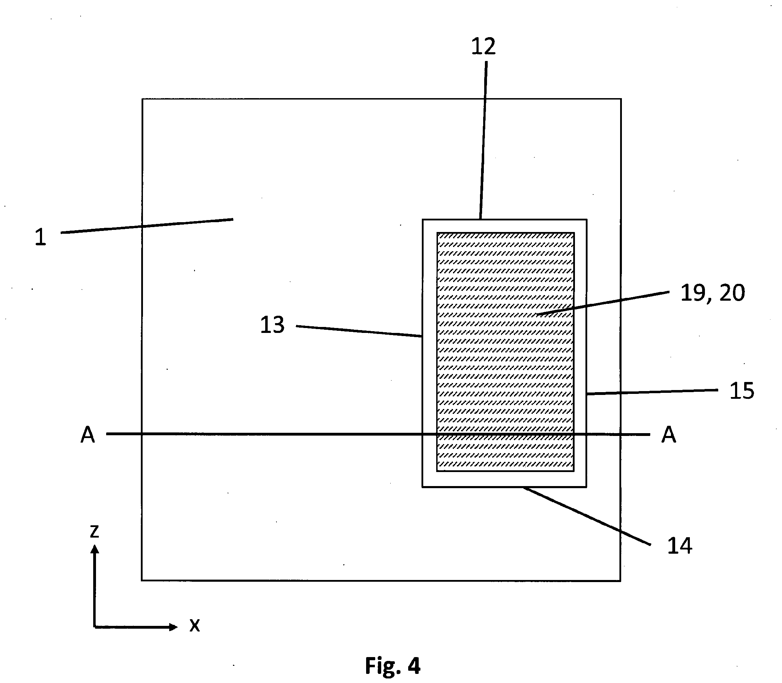

Der Querschnitt A-A durch die beispielhafte Ausführungsform des Gehäuses 1 aus Figur 2 ist in Figur 3 zu sehen. Beispielsweise ist in der gezeigten Ausführungsform die Aufnahme 7 mit der Schnittstelle 6 so ausgeführt, dass diese leicht in den Raum der Vertiefung hineinragen und am Boden 8 angeordnet sind. Durch die gestrichelte Linie bei der Öffnung 11 wird die Oberkante einer der Umfangswände 12 bzw. 14 angedeutet. Das Querschnittsprofil entlang der Richtung von der Öffnung 11 zum Boden 8 (y-Richtung) ist beispielsweise rechteckig. Darüber hinaus sind aber auch andere Querschnittsprofile der Vertiefung denkbar. An den Umfangswänden 12, 13, 14, 15 sind Führungsmittel angebracht, welche einen Datenübertragungsstick 19 oder einen Datenspeicherstick 20 beim Einführen führen und im montierten Zustand, also in die Schnittstelle 6 eingesteckt, stützen. Bei Benutzung der Schnittstelle 6 ist ein Datenübertragungsstick 19 oder ein Datenspeicherstick 20 mit der Schnittstelle 6 verbunden, wie in Figur 4 beispielhaft gezeigt ist. Durch die Verbindung von Datenübertragungsstick 19 mit der Schnittstelle 6 wird beispielsweise eine Sende/Empfangsanlage dargestellt, welche mit zum Beispiel einem Ethernet- und/oder Funk- und/oder GSM- und /oder UMTS- und/oder 4G- und/oder 5G-Netz verbunden werden kann. Die Vertiefung 9 ist dabei so dimensioniert, dass ein Datenübertragungsstick 19 oder ein Datenspeicherstick 20 vollständig von der Vertiefung aufgenommen werden kann. In manchen Ausführungsformen ist es zudem möglich, dass die Vertiefung 9 so dimensioniert ist, dass der Datenübertragungsstick 19 oder der Datenspeicherstick 20 teilweise durch die Öffnung 11 über die Oberfläche 31 des Gehäuses hinausragt.

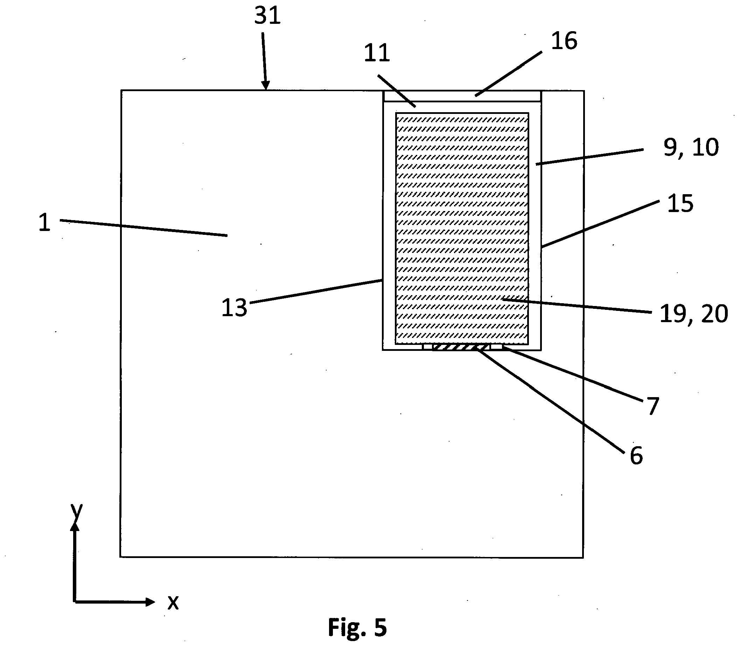

In Figur 5 ist der Querschnitt A-A des beispielhaften Gehäuses 1 aus Figur 4 gezeigt. Ein Datenübertragungsstick 19 oder ein Datenspeicherstick 20 ist mit in der Aufnahme 7 mit der Schnittstelle 6 verbunden. Die Vertiefung 9 ist beispielsweise so dimensioniert, dass der eingesetzte Datenübertragungsstick 19 bzw. Datenspeicherstick 20 nicht über die Oberfläche 31 des Gehäuses 1 hinausragt. Die Vertiefung 9 im Bereich der Öffnung 11 ist mit Deckel 16 verschlossen. In manchen Ausführungsformen kann der Deckel 16 beispielsweise auch flächenbündig mit dem Gehäuse 1 abschließen. Der Deckel 16 ist beispielsweise unverlierbar mit dem Gehäuse 1 verbunden. Eine solche Verbindung kann beispielsweise durch ein Scharnier 26 erreicht werden. Auch eine einfache Verbindung über eine Art Band zwischen Gehäuse 1 und Deckel 16 kann eine unverlierbare Verbindung realisieren. In einigen beispielhaften Ausführungsformen ist der Deckel 16 fest auf der Vertiefung 9 verschließbar. Beispielsweise kann dem Deckel 16 oder dem Gehäuse 1 ein Schloss angeordnet sein, welches den Deckel 16 mit einem Schlüssel verschließen lässt. Ein solcher Verschluss stellt eine beispielhafte Maßnahme dar, damit der Datenübertragungsstick 19 oder der Datenspeicherstick 20 vor einer unautorisierten Entnahme geschützt wird. Alternativ kann der Deckel 16 beispielsweise auch mit anderen Werkzeugen verschließbar ausgeführt sein. Beispielsweise kann das Einschrauben einer Schraube in entsprechende Vorrichtungen in Deckel 16 und Gehäuse 1 den Deckel 16 fest auf der Vertiefung 9 verschließen.

Auch kann der Deckel 16 zum Beispiel so mit der Aufnahme 7 und Schnittstelle 6 gekoppelt sein, dass beim Öffnen des Deckels 16 die Aufnahme mitsamt Schnittstelle 6 in Richtung der Öffnung 11 bewegt wird. Beispielsweise ragt dadurch bei vollständig geöffnetem Deckel 16 ein in die Schnittstelle 6 gesteckter Datenübertragungsstick 19 oder Datenspeicherstick 20 mindestens teilweise über die Gehäuseoberfläche 31 hinaus.

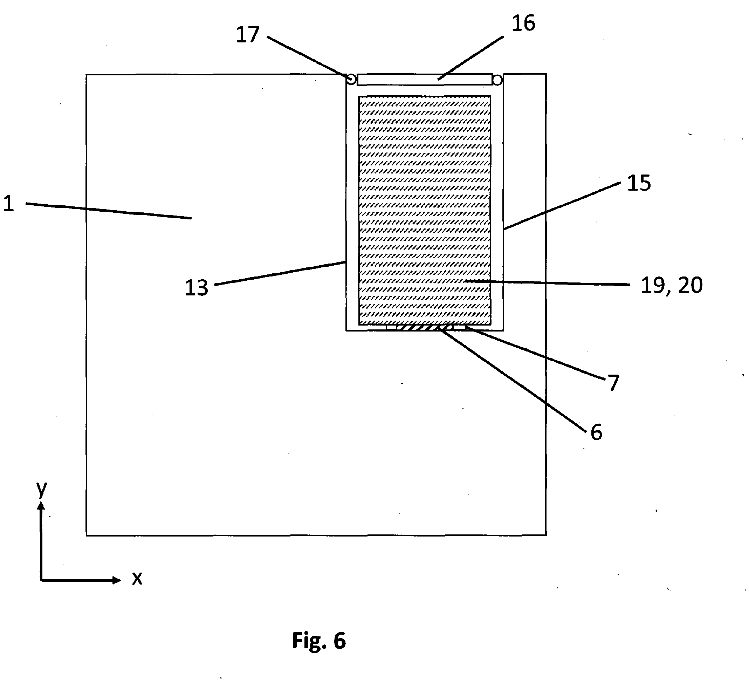

Eine beispielhafte Ausführungsform des Gehäuses 1 mit Deckel 16 ist auch in Figur 6 dargestellt. Der Deckel 16 ist hier beispielhaft mit umlaufenden Dichtungen 17 ausgestattet. Mit Hilfe dieser Dichtungen 17 kann beispielsweise ein Spritzwasser- und Berührungsschutz der Schnittstelle 6 erreicht werden. Die Dichtung 17 ist hier beispielhaft als Teil des Deckels 16 dargestellt, kann aber auch als Teil der Öffnung 11 bzw. allgemein als Teil der Vertiefung 9 angeordnet sein. Beispielsweise sind Deckel 16 und Vertiefung 9 so aufeinander abgestimmt, dass eine am Deckel 16 oder an der Vertiefung 9 angeordnete Dichtung 17 dazu beiträgt, dass die Vertiefung 9 und damit auch die Schnittstelle 6 gegenüber des Äußeren des Beatmungsgeräts abgedichtet sind. In Figur 6 ist die Dichtung 17 beispielhaft zwischen den Umfangswänden 12, 13, 14, 15 und dem Deckel 16 angeordnet. Denkbar sind aber auch Anordnungen der Dichtung 17 beispielsweise auf bzw. einem Rand bzw. Kragen entlang der Umfangswände, auf bzw. in dem die Dichtung 17 fest eingesetzt ist. Bei Verschließen des Deckels 16 liegt dieser dann auf der umlaufenden Dichtung 17 auf bzw. wird beispielsweise auf die Dichtung 17 aufgedrückt. In anderen beispielhaften Ausführungsformen kann auch an der Unterseite des Deckels 16 ein gewisser Absatz angeordnet sein, um den die Dichtung 17 verläuft. Bei Verschließen des Deckels 16 drückt diese Dichtung dann beispielsweise gegen die Umfangswände der Vertiefung 9 oder werden auf einen etwaigen Rand bzw. Kragen, welcher umlaufend entlang der Umfangswände angeordnet ist, aufgedrückt. Alternativ kann auch der Deckel 16 selbst als Dichtung 17 ausgeführt sein. Dazu besteht der Deckel beispielsweise aus einem weichen Kunststoff, beispielsweise Silikon, und lässt sich abdichtend in die Öffnung 11 der Vertiefung drücken. In einer beispielhaft flächenbündig abschließenden Ausführung des Deckels 16 könnte beispielsweise eine als Lasche ausgebildete Armatur 23 an dem Deckel 16 angebracht sein, um daran den Deckel aus der Öffnung 11 herausziehen zu können. Die Vertiefung 9 ist beispielsweise so dimensioniert, dass der Datenübertragungsstick 19 bzw. Datenspeicherstick 20 vom Deckel 16 beabstandet eingesetzt werden kann. Dieser Abstand zwischen Datenübertragungsstick 19 bzw. Datenspeicherstick 20 und Deckel 16 sollte mindestens so groß sein, dass der Deckel 16 keine mechanische Belastung auf den Datenübertragungsstick 19 bzw. den Datenspeicherstick 20 ausübt. Auch ein augenscheinlich nicht vorhandener Abstand zwischen Deckel 16 und Datenübertragungsstick 19 bzw. Datenspeicherstick 20 ist möglich. In manchen Ausführungsformen drückt beispielsweise der Deckel 16 leicht auf den Datenübertragungsstick 19 bzw. den Datenspeicherstick 20, sodass dieser im Gehäuse gestützt wird. Beispielsweise ist an dem Deckel und/oder im Schacht Halterungselement angebracht, in welches der beispielsweise Datenübertragungsstick 19 eingesetzt wird, sodass dieser festgeklemmt ist oder eingepresst wird. Beispielsweise kann dieses Halterungselement ein elastisches Schaumstoffelement sein, welches zum Beispiel auf der Innenseite des Deckels 16 und/oder in dem Schacht 10 angebracht ist. Ist die Schnittstelle 6 beispielsweise am Boden 8 des Schachtes 10 angeordnet, so kann beispielsweise an der Innenseite des Deckels 16 ein Schaumstoffelement angebracht sein. Das Schaumstoffelement ist dabei so ausgeführt, dass, wenn der Deckel 16 bei eingestecktem Datenübertragungsstick 19 bzw. Datenspeicherstick 20 geschlossen wird, der Deckel 16 bzw. das Schaumstoffelement auf den Datenübertragungsstick 19 bzw. den Datenspeicherstick 20 drückt. Dabei verformt sich das beispielhafte Schaumstoffelement so, dass es im Wesentlichen dem Datenübertragungsstick 19 bzw. dem Datenspeicherstick 20 nachgibt, dadurch aber andererseits auch zu Fixierung beiträgt. Statt einem Schaumstoffelement kann beispielsweise auch ein Federelement an der Innenseite des Deckels 16 angebracht sein, welches ebenfalls die Wirkung eines eher leichten Drucks auf einen eingesteckten Datenübertragungsstick 19 bzw. Datenspeicherstick 20 zur Fixierung erzielen kann.

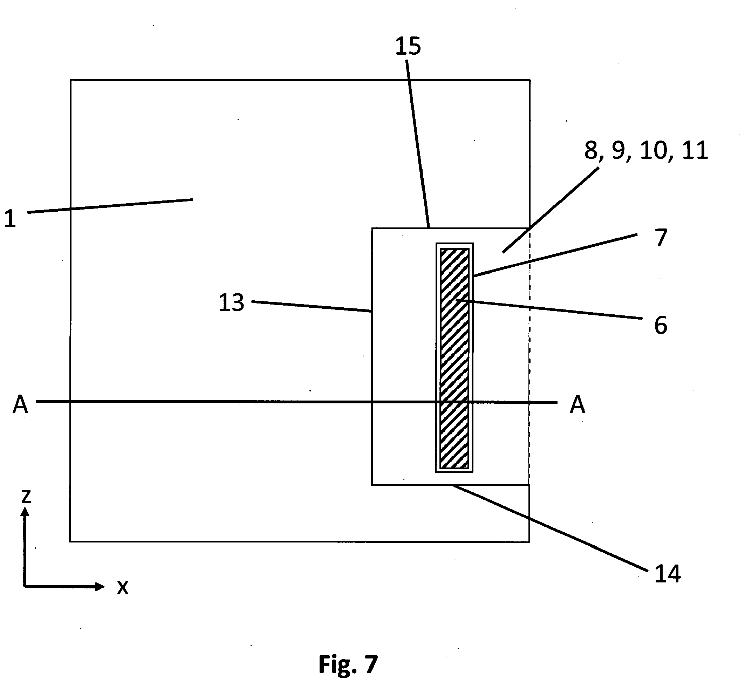

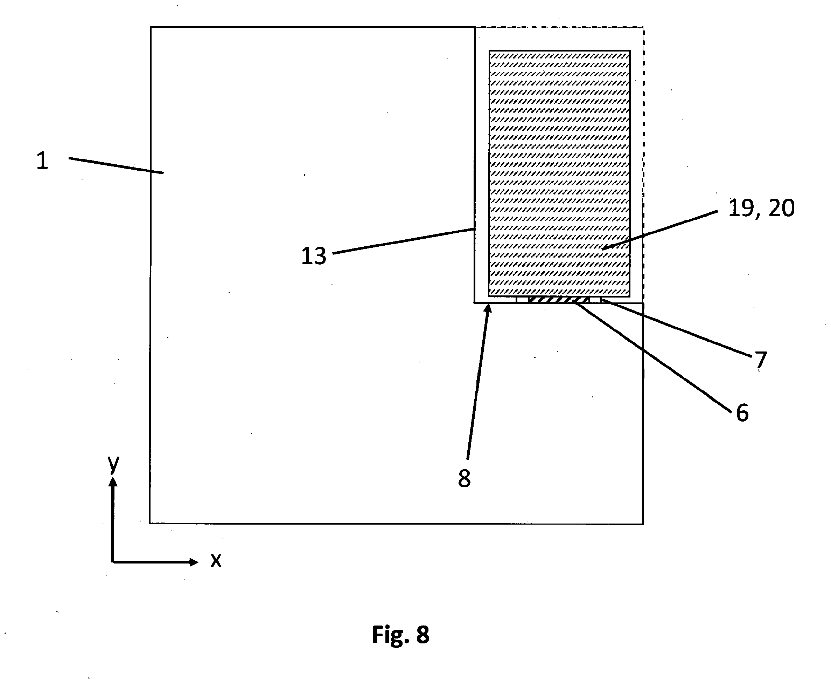

In manchen beispielhaften Ausführungsformen ist die Vertiefung 9 zumindest teilweise in einer Außenfläche 30 des Gehäuserandes 18 angeordnet. Eine solche beispielhafte Ausführungsform ist in Figur 7 in Draufsicht und in Figur 8 im Querschnitt A-A der Figur 7 gezeigt. Durch die Lage der Vertiefung im Gehäuserand 18 ist die Vertiefung 9 neben der Öffnung 11 zur Oberfläche 31 des Gehäuses 1 auch zu einer Außenfläche 30 im Gehäuserand 18 offen. Steckt, wie in Figur 8 beispielhaft dargestellt, ein Datenübertragungsstick 19 oder ein Datenspeicherstick 20 in der Schnittstelle 6, ist dieser von zwei Seiten zugänglich.

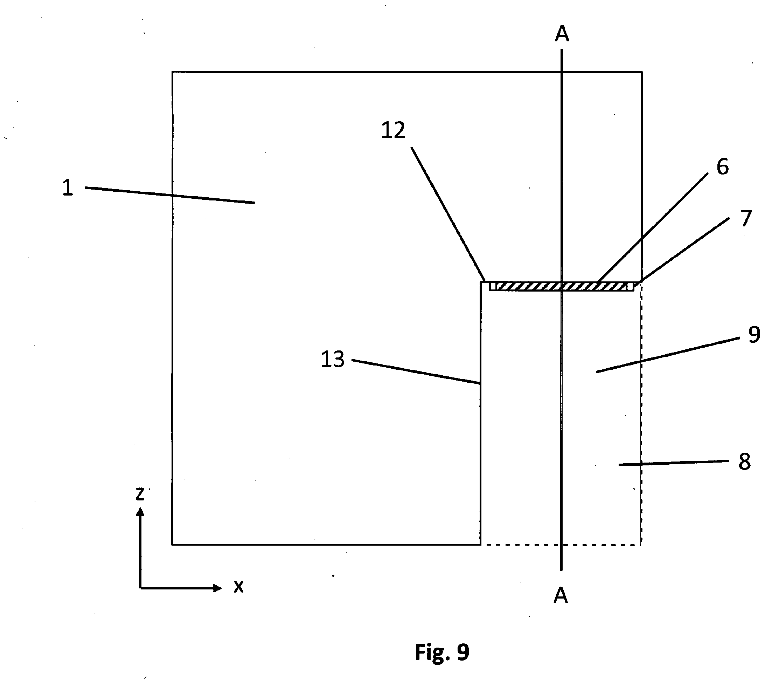

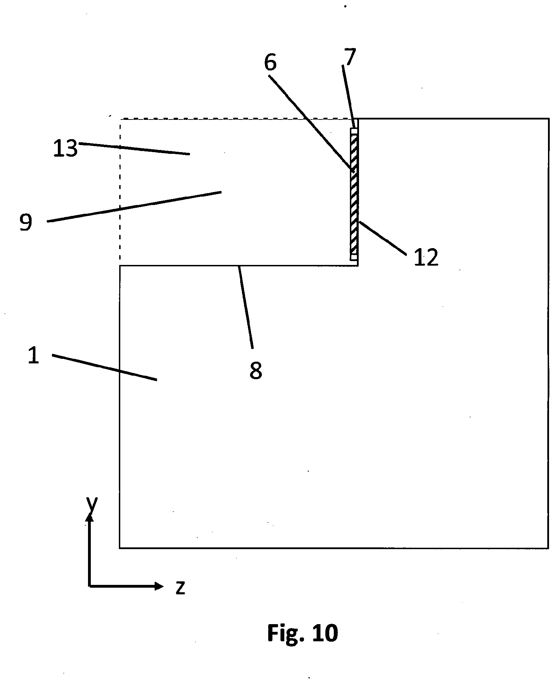

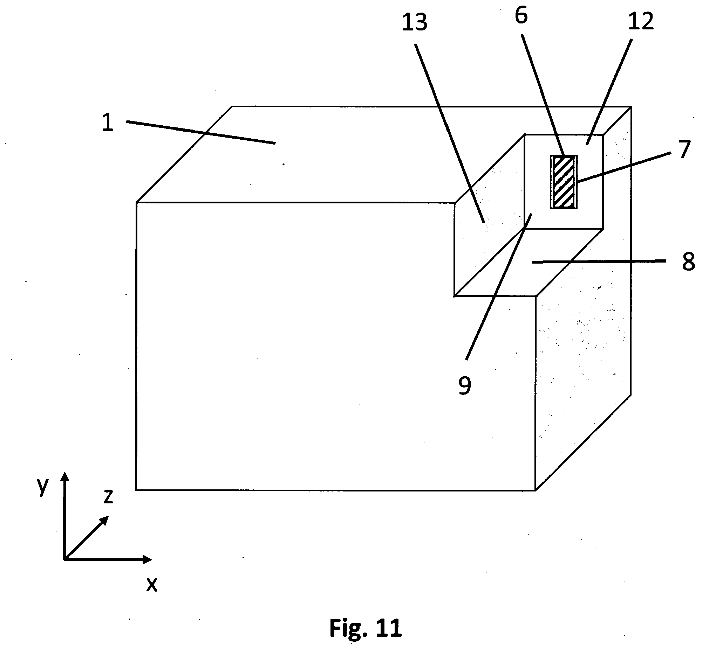

Bei der in Figuren 9, 10 und 11 dargestellten beispielhaften Ausführungsformen des Gehäuses 1 ist die Vertiefung 9 in einer Ecke des Gehäuses 1 angeordnet und ist somit Teil von zwei Außenflächen 31 des Gehäuserandes 18. Die Figur 9 zeigt dabei eine Draufsicht des Gehäuses 1, dessen Querschnitt A-A in Figur 10 schematisch dargestellt ist. Zur Verdeutlichung der Lage der Vertiefung 9 in einer Ecke des Gehäuses 1 ist das Gehäuse 1 stark vereinfacht in Figur 11 in einer perspektivischen Ansicht dargestellt. In der in Figuren 9 bis 11 gezeigten Ausführungsform ist die Aufnahme 7 mit der Schnittstelle 6 in der Umfangswand 12 angeordnet. Genauso ist auch eine Anordnung der Aufnahme 7 mit der Schnittstelle 8 in einer anderen Umfangswand oder dem Boden 8 möglich. Um einen Spritzwasser- und/oder Berührungsschutz herzustellen wird ein Deckel 16 an der Vertiefung 9 angebracht, sodass alle drei offenen Seiten verschlossen werden.

Der Deckel 16 kann dabei beweglich mit dem Gehäuse 1 unverlierbar verbunden sein oder auch vollständig abnehmbar ausgeführt sein. Beispielsweise ist der Deckel 16 vollständig abnehmbar ausgeführt und kann beim Verschließen der Vertiefung 9 mit entsprechenden Vorrichtungen im Gehäuse 1 und am Deckel 16 lösbar verbunden werden, beispielsweise durch einrasten oder andere Mittel und Wege. In einer anderen beispielhaften Ausführungsform kann der Deckel 16 auch an einer Seite über ein Scharnier 26 mit dem Gehäuse 1 verbunden sein. In weiteren Ausführungsformen ist die unverlierbare Verbindung zwischen Gehäuse 1 und Deckel 16 durch eine Art Band realisiert.

Bei einer Lage der Aufnahme 7 mit der Schnittstelle 6 in einer der Umfangswände 12, 13, 14, 15 der Vertiefung 9 kann beispielsweise auf der gegenüberliegenden Seite - im Deckel 16 oder der gegenüberliegenden Umfangswand - ein oder mehrere Federelemente angebracht werden um die Halterung des Datenübertragungssticks 19 bzw. des Datenspeichersticks 20 in der Schnittstelle 6 zu unterstützen. Beim Einsetzen beispielsweise eines Datenübertragungssticks 19 werden die Federelemente beispielsweise zunächst durch den Datenübertragungsstick 19 zusammengedrückt, sodass ein Einstecken des Datenübertragungssticks 19 in die Schnittstelle 6 ermöglicht wird. Beim Einstecken des Datenübertragungssticks 19 in die Schnittstelle 6 dehnen sich die Federelemente dabei wieder teilweise aus, erreichen aber nicht ihre ursprüngliche Ausdehnung. Durch das weitere Bestreben der Federelemente wieder die ursprüngliche Ausdehnung anzunehmen, wird der beispielsweise Datenübertragungsstick 19 zusätzlich in die Schnittstelle 6 gedrückt und dadurch gehaltert. Eine Kombination aus verschiedenen Halterungs- und Fixiermethoden des Datenübertragungssticks 19 oder des Datenspeichersticks 20 in der Schnittstelle 6 bzw. der Vertiefung 9 sind dabei ebenfalls denkbar. So können beispielsweise die hier beschriebenen Federelemente in der der Schnittstelle 6 gegenüberliegenden Umfangswand 12, 13, 14, 15 angeordnet sein und zusätzlich auch ein oder mehrere Schaumstoffelemente an der Innenseite des Deckels 16 angebracht sein.

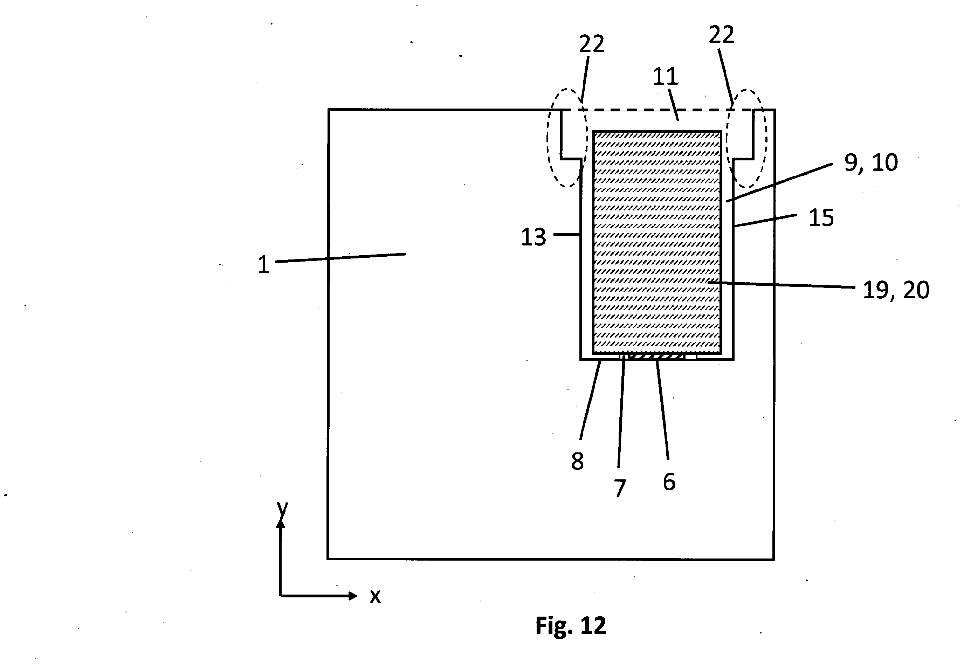

In Figur 12 ist eine beispielhafte Ausführungsform des Gehäuses 1 des Beatmungsgeräts schematisch gezeigt, wobei die Vertiefung 9 über Eingriffsbereiche 22 verfügt. Diese Eingriffsbereiche 22 sind beispielsweise durch eine Verbreiterung der Vertiefung 9 im Bereich der Öffnung 11 realisiert. Dadurch ist der Bereich der Öffnung 11 gegenüber dem als Schacht 10 ausgeführten Teil der Vertiefung 9 so aufgeweitet, dass ein Umgreifen des Datenübertragungssticks 19 oder eines Datenspeichersticks 20 mit einem oder mehr Fingern und/oder Daumen möglich ist. Beispielsweise sind diese Eingriffsbereiche 22 so dimensioniert, dass beispielsweise ein Datenübertragungsstick 19 zum Teil über die Kante zwischen Umfangswand 12, 13, 14, 15 der Vertiefung 9 und dem Eingriffsbereich 22 ragt. Beispielsweise können die Eingriffsbereiche 22 auch mit weiteren Funktionen verknüpft werden wie zu Beispiel Vorrichtungen zum Verschließen des Deckels 16.

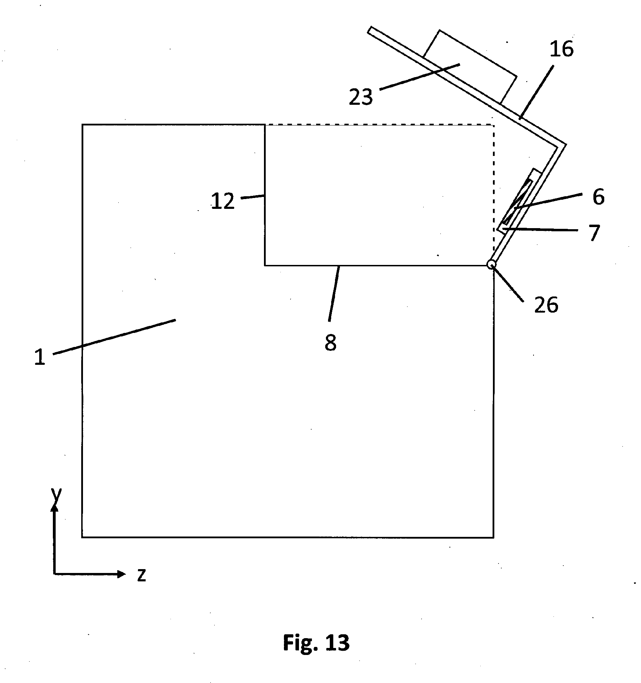

Neben einer Anordnung der Aufnahme 7 mit der Schnittstelle 6 in dem Boden 8 oder einer der Umfangswände 12, 13, 14, 15 der Vertiefung 9 ist beispielsweise auch eine Anordnung der Aufnahme 7 mit Schnittstelle 6 im Deckel 16 möglich. Eine solche nicht von der beanspruchten Erfindung erfasste beispielhafte Ausführungsform ist in Figur 13 dargestellt. Der Deckel 16 ist dabei zum Beispiel über ein Scharnier 26 mit dem Gehäuse 1 verbunden. Die Aufnahme 7 mit der Schnittstelle 6 ist ein einer Seite des Deckels 16 angebracht. So kann beispielsweise der Datenübertragungsstick 19 oder der Datenspeicherstick 20 in den geöffneten Deckel 16 eingesteckt werden und wird durch Schließen des Deckels 16 in die Vertiefung 9 geführt. Beispielsweise ist der Deckel 16, genauer die im Deckel 16 angeordnete Schnittstelle 6 nicht nur mechanisch, sondern auch elektrisch mit dem Gehäuse 1 bzw. dem Beatmungsgerät verbunden. Bei fehlender elektrischer Verbindung zwischen Deckel 16 bzw. Schnittstelle 6 mit dem Beatmungsgerät würde demnach die Schnittstelle 6 funktionslos werden, da Daten zwischen Datenübertragungsstick 19 bzw. Datenspeicherstick 20 und dem Beatmungsgerät nicht ausgetauscht werden könnten. Beispielsweise ist der Deckel 16 unverlierbar bzw. permanent aber beweglich mit dem Gehäuse 1 verbunden. In einer beispielhaften Ausführungsform, bei der Deckel 16 über eine Art Band mit dem Gehäuse verbunden ist, kann dieses Band auch gleichzeitig die Funktion eines Kabels zur elektrischen Verbindung zwischen Deckel 16 und Beatmungsgerät übernehmen. Der Deckel 16 kann auch separat, also nicht unverlierbar mit dem Gehäuse 1 verbunden, ausgeführt werden. Befindet sich die Aufnahme 7 mit der Schnittstelle 6 dabei beispielsweise im/am Deckel 16, so müssen Deckel 16 und Gehäuse 1 bzw. das Beatmungsgerät Vorrichtungen aufweisen, sodass eine elektrische Verbindung zwischen Deckel 16 und Beatmungsgerät hergestellt werden kann, wenn der Deckel 16 angebracht bzw. geschlossen wird.

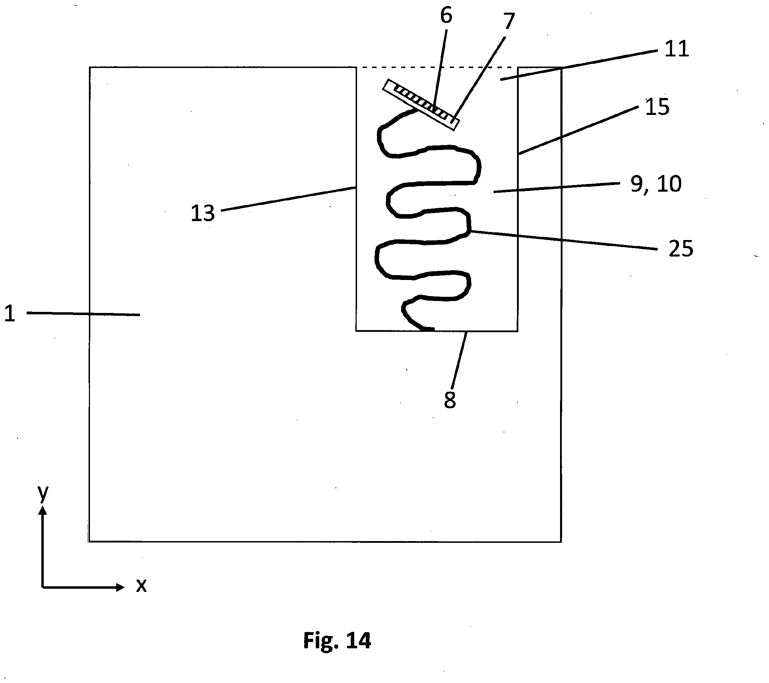

Beispielsweise verfügt der Deckel 16 auch über eine Armatur 23. Diese Armatur 23 kann beispielsweise in Form eines Stegs oder eines dünnen Vorsprungs an der Außenseite des Deckels 16 - also an der Seite, welche bei geschlossenem Deckel 16 nicht in der Vertiefung 9 liegt - angebracht sein. Eine solche Armatur kann beispielsweise dazu genutzt werden, gegriffen zu werden um den Deckel 16 zu öffnen. In Figur 14 wird eine beispielhafte Ausführungsform des Beatmungsgeräts schematisch dargestellt, bei dem die Aufnahme 7 mit Schnittstelle 6 an einem Kabel 25 in der Vertiefung 9 angeordnet ist. Das Kabel 25 ist so ausgeführt, dass die Aufnahme 7 mit Schnittstelle 6 zumindest teilweise durch die Öffnung 11 aus der Vertiefung 9 gezogen werden kann um einen Datenübertragungsstick 19 oder einen Datenspeicherstick 20 mit der Schnittstelle 6 zu verbinden bzw. wieder zu entnehmen.

Beispielsweise verfügt die Vertiefung 9 zusätzlich über eine Vorrichtung, welche einen mit der Schnittstelle 6 verbundenen Datenübertragungsstick 19 oder einen Datenspeicherstick 20 in der Vertiefung 9 haltert. Dadurch können zum Beispiel unbeabsichtigte Bewegungen des Datenübertragungssticks 19 bzw. des Datenspeichersticks 20, welche eventuell auch zu einem unbeabsichtigten Trennen von der Schnittstelle führen können, verhindert werden. Beispielsweise wird das Kabel 25 so durch den Boden 8 in den Innenraum des Beatmungsgeräts geführt, dass der Boden um das Kabel 25 so abgedichtet ist, dass der der Übergang des Kabels 25 in den Innenraum des Beatmungsgeräts einen Spritzwasser- und Berührungsschutz aufweist.

In einer alternativen oder ergänzenden Ausführungsform kann auch eine Durchführung 35 im Boden 8 der Vertiefung 9 ausgebildet sein, durch welche eine Leitung des Datenübertragungssticks 19 geführt wird, welche mit einer Schnittstelle 6 bzw. Steckverbindung 36 direkt auf einer elektronischen Leiterplatte 37 des Beatmungsgerätes verbunden wird.

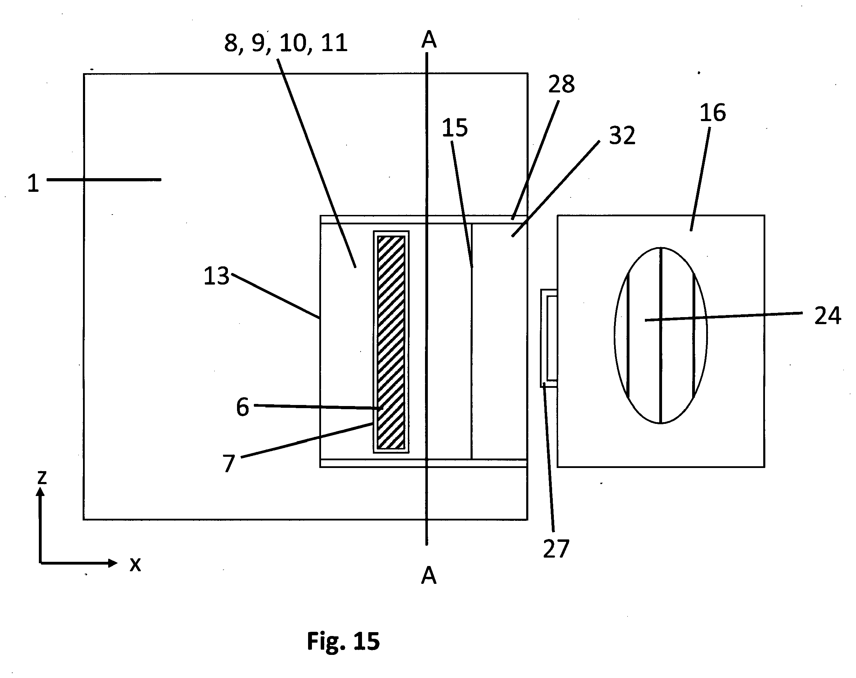

Der Deckel 16 kann auch zum Beispiel als Gleitschieber ausgeführt sein, wie in einer beispielhaften Ausführungsform in Figur 15 gezeigt. In der gezeigten Ausführungsform erstreckt sich die Öffnung 11 beispielhaft nur in der Oberfläche 31 des Gehäuses 1. Der als Gleitschieber ausgeführte Deckel 16 wird dabei auf zumindest einer Führungsschiene 28, welche im Bereich der Öffnung 11 an zumindest einer Umfangswand 12, 13, 14, 15 angebracht ist, geführt. Beispielweise ist der Deckel 16 mit einer Verschlussvorrichtung 27 ausgestattet, zu welcher ein nicht näher gezeigtes und beschriebenes Gegenstück im Gehäuse 1 angebracht ist. Die Verschlussvorrichtung 27 bewirkt dabei, dass der Deckel 16 im verschlossenen Zustand fest auf der Öffnung 11 sitzt. Die Verschlussvorrichtung 27 kann beispielsweise so eingerichtet sein, dass eine elektronische Verriegelung einsetzt, falls ein Datenübertragungsstick 19 oder eine Datenspeicherstick 19 in die Schnittstelle 6 gesteckt wird und der Deckel 16 geschlossen wird. Ist der Deckel 16 beispielsweise so eingerichtet, dass dieser flächenbündig mit der Gehäuseoberfläche 31 abschließt, wobei die Vertiefung 9 beabstandet zum Gehäuserand 18 angeordnet ist, so ist beispielsweise ein Absatz 32 zu einem der Gehäuseränder 18 ausgebildet, welcher gegenüber der Gehäuseoberfläche 31 abgesenkt ist. Dieser Absatz 32 ist so abgesenkt, dass der Deckel 16 auf den Führungsschienen 28 über die Öffnung 11 geschoben werden kann und dabei flächenbündig mit der Gehäuseoberfläche abschließt.

In einer beispielhaften Ausführungsform ist auf dem Deckel 16 eine Oberflächenstrukturierung 25 aufgebracht. Diese Oberflächenstrukturierung ist beispielsweise so ausgeführt, dass der Gleitwiderstand zwischen beispielsweise den Fingern und dem Deckel 16 erhöht wird.

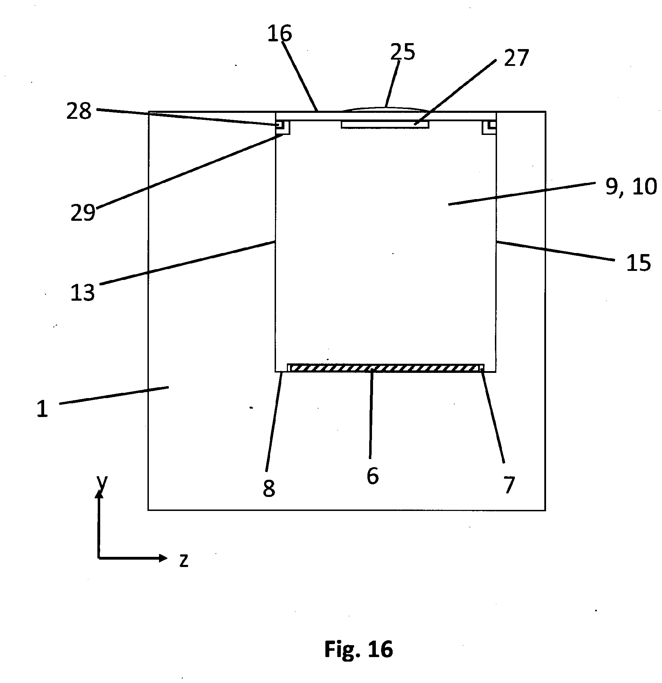

In Figur 16 ist der Querschnitt A-A durch die beispielhafte Ausführungsform des Gehäuses 1 mit dem als Gleitschieber ausgeführten Deckel 16 zu sehen. Beispielsweise sind an den Umfangswänden 13 und 15 Führungsschienen 28 angebracht. Zur Positionierung des Deckels 16 sind am Deckel 16 passende zu den Führungsschienen 28 Gleitelemente 29 angebracht. Die Gleitelemente 29 können zusätzlich auch eine halternde Funktion des Deckels leisten, z.B. wenn die Vertiefung 9 auf der Unterseite des Beatmungsgeräts angebracht ist und der Deckel 16 gegen herausfallen gesichert sein soll.

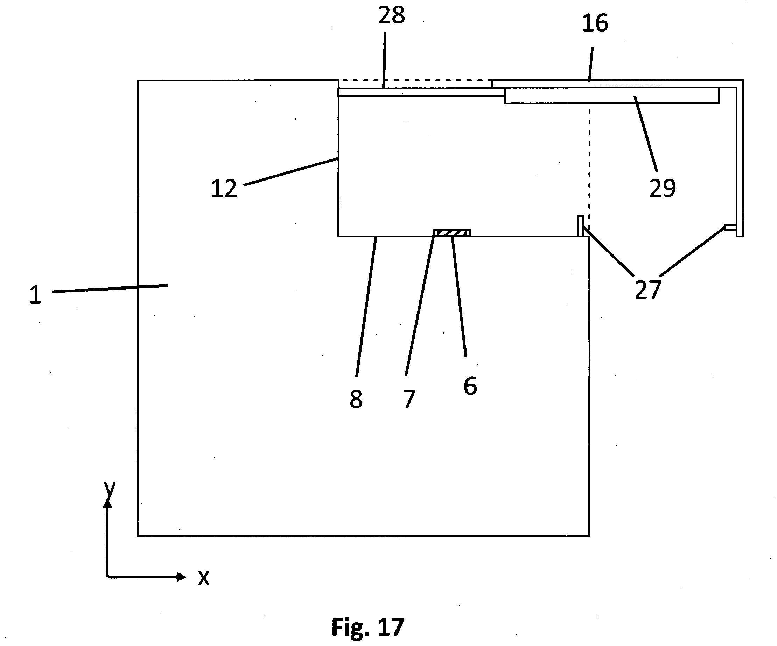

Figur 17 zeigt eine beispielhafte Ausführungsform des Gehäuses 1 und des Deckels 16, wobei die Vertiefung 9 im Gehäuse 1 Teil einer der Gehäuseränder 18 ist und sich teilweise über eine Außenfläche 30 erstreckt. An zwei sich gegenüberliegenden Umfangswänden sind Führungsschienen 28 angebracht, mit denen der Deckel 16 über die Gleitelemente 29 geführt werden können. Dazu sind an dem Deckel 16 und beispielsweise am Boden 8 der Vertiefung 9 Verschlussvorrichtungen 27 angebracht, welche zusammen beispielsweise ein Verriegeln des Deckels 16 mit dem Gehäuse 1 ermöglichen. Die Verriegelung kann beispielsweise automatisch ausgelöst werden, wenn der Deckel 16 geschlossen wird und ein Datenübertragungsstick 19 oder ein Datenspeicherstick 20 mit der beispielhaft am Boden 8 angeordneten Schnittstelle 6 verbunden ist. Eine Öffnung der Verriegelung wäre dazu beispielsweise durch eine Authentifizierung am Beatmungsgerät möglich. Auch ein einfaches Einrasten der Verschlussvorrichtung 27 des Gehäuses 16 in der Verschlussvorrichtung 27 - oder anders herum - ist beispielsweise denkbar. Dabei könnte der Deckel 16 beispielsweise auch entfernt werden, ohne dass eine Authentifizierung beispielsweise am Beatmungsgerät nötig ist. In anderen beispielhaften Ausführungsformen sind die Verschlussvorrichtungen 27 so ausgebildet, dass zum Beispiel mit Hilfe eines Schlüssels der Deckel 16 verschlossen bzw. verriegelt werden kann.

Auch die Verwendung, beispielsweise die Inbetriebnahme eines Datenübertragungssticks 19 nach Verbindung mit der Schnittstelle 6, kann eine Authentifizierung erfordern. Wird ein Datenübertragungsstick 19 mit der Schnittstelle 6 verbunden, kann der Stick 19 beispielsweise erst dann mit einer Datenübertragung beginnen, wenn dies über das Beatmungsgerät freigegeben bzw. authentifiziert wird. In manchen Ausführungsformen reicht dabei eine einmalige Freigabe nach der Verbindung Datenübertragungsstick 19 mit Schnittstelle 6 aus.

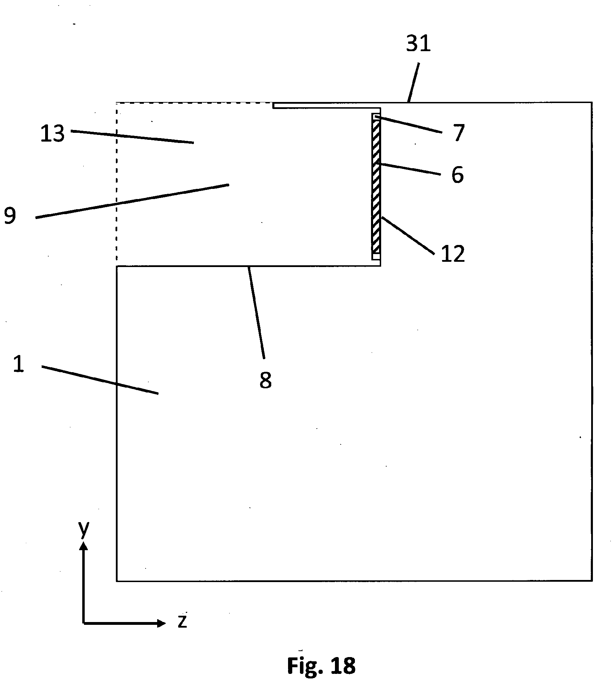

Ist die Aufnahme 7 mit der Schnittstelle 6 beispielsweise in einer der Umfangswände 12, 13, 14, 15 angeordnet, wie in Figur 18 beispielhaft dargestellt, kann ein Teilbereich der Vertiefung 9 von der Gehäuseoberfläche 31 verdeckt sein. Beispielsweise kann der Bereich der Vertiefung 9, in dem die Aufnahme 7 mit der Schnittstelle 6 in einer der Umfangswände angeordnet sind, durch die Gehäuseoberfläche 31 verdeckt werden.

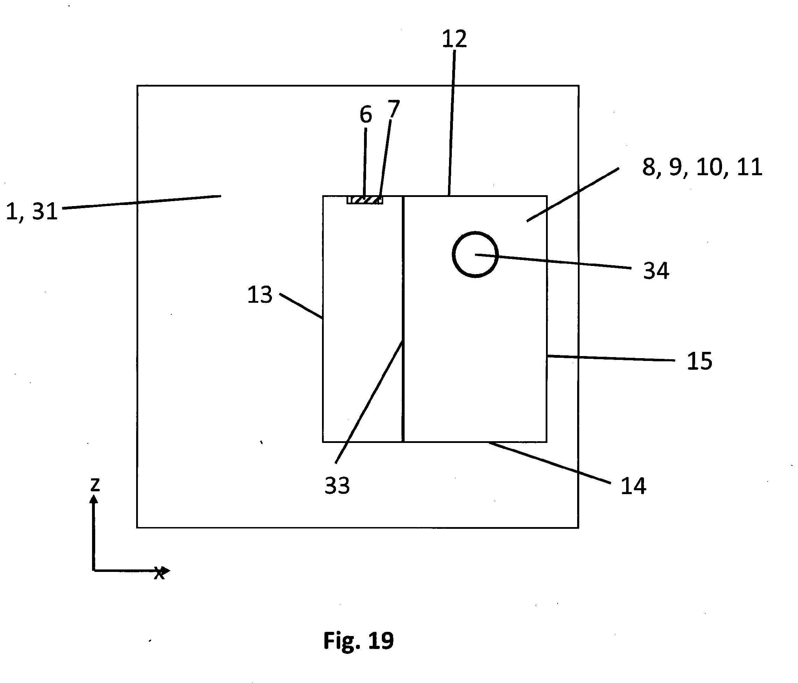

Figur 19 zeigt eine beispielhafte Ausführungsform des Gehäuses 1 bei dem in der Vertiefung 9 neben der Aufnahme 7 mit der Schnittstelle 6 beispielsweise noch ein weiterer Anschluss 34 angeordnet ist. Die Aufnahme 7 mit der Schnittstelle 6 ist in der in Figur 19 gezeigten Ausführungsform beispielhaft an der Umfangswand 12 angeordnet, kann aber auch in einer der anderen Umfangswände 13, 14, 15, dem Boden 8 oder den hier nicht gezeigten Deckel 16 angeordnet sein. Ebenso können der weitere Anschluss 34 oder noch mehr zusätzlich Anschlüsse oder Bauteile in dem Boden 8 oder zumindest einer der Umfangswände 12, 13, 14, 15 oder dem Deckel 16 angebracht sein. Der Anschluss 34 kann beispielsweise so ausgebildet sei, dass eine O₂-Messzelle angeschlossen werden kann. Auch andere Funktionen des Anschlusses 34, wie beispielsweise zum Anschließen eines Akkus oder anderer Analyse- oder Medizingeräte ist möglich. Die Anzahl und Funktion weiterer Anschlüsse 34 oder auch nicht näher beschriebener Bauteile ist dabei durch die genannten Beispiele nicht begrenzt. Es ist also zu verstehen, dass neben der beschriebenen Schnittstelle 6 in Anzahl und Funktion nicht begrenzte weitere Bauteile und Anschlüsse in der Vertiefung 9 angeordnet sein können. Beispielsweise ist der Bereich der Schnittstelle 6 durch eine Zwischenwand 33 von dem Bereich mit den zusätzlichen Anschluss 34 zumindest teilweise räumlich getrennt. Beispielweise kann die Zwischenwand 33 eine Höhe haben, sodass die Zwischenwand 33 mit der Gehäuseoberfläche 31 bündig abschließt. In einigen Ausführungsformen ist die Höhe der Zwischenwand 33 beispielweise so gewählt, dass die Zwischenwand 33 nicht bündig mit der Gehäuseoberfläche 31 abschließt. Beispielsweise weist die Zwischenwand 33 Mittel auf bzw. ist ausgebildet um gegen hochfrequente Wellen sowie elektrische Felder abzuschirmen. Beispielsweise kann die Zwischenwand 33 auch Mittel aufweisen bzw. ausgebildet sein, sodass elektrische Felder und Quellen nur in eine Vorzugsrichtung ermöglicht werden. Bei einer Anordnung von mehreren Anschlüssen 34 und/oder Bauteilen neben der Schnittstelle 6 können auch weitere Zwischenwände 33 die jeweiligen Bereiche voneinander zumindest teilweise räumlich trennen.

Eine beispielhaft alternative Ausführung der in Figur 19 dargestellten Ausführungsform umfasst statt einer Schnittstelle 6 mit Aufnahme 7 direkt in der Vertiefung 9 eine Durchführung 35 für eine Leitung (beispielsweise ein Kabel) vom Datenübertragungsstick 19 zu einem Ansteckpunkt 36 der Schnittstelle 6 auf einer elektronische Leiterplatte 37 (nicht dargestellt) des Beatmungsgerätes. Die Durchführung 35 ist beispielsweise so angeordnet und eingerichtet, dass der Ansteckunkt 36 durch die Durchführung 35 erreichbar ist, der Datenübertragungsstick 19 in der Vertiefung 9 platzierbar ist und der Datenübertragungsstick 19 über eine Leitung mit dem Ansteckpunkt 36 der Schnittstelle 6 verbindbar ist. In manchen Ausführungsformen kann auch eine Leitung mit dem Ansteckpunkt 36 der Schnittstelle 6 verbunden sein, sodass die Leitung eine Verlängerung der Schnittstelle 6 darstellt, wie beispielhaft durch das Kabel 25 in Figur 14 dargestellt.

### Bezugszeichenliste

- 1: Gehäuse
- 2: Atemgasquelle
- 3: Steuersystem
- 4: Steuersystem
- 5: Steuersystem
- 6: Schnittstelle
- 7: Aufnahme
- 8: Boden
- 9: Vertiefung
- 10: Schacht
- 11: Öffnung
- 12: Umfangswand
- 13: Umfangswand
- 14: Umfangswand
- 15: Umfangswand
- 16: Deckel
- 17: Umlaufende Dichtung
- 18: Gehäuserand
- 19: Datenübertragungsstick
- 20: Datenspeicherstick
- 21: Entnahmevorrichtung
- 22: Eingriffsbereich
- 23: Armatur
- 24: Oberflächenstrukturierung
- 25: Kabel
- 26: Scharnier
- 27: Verschlussvorrichtung
- 28: Führungsschiene
- 29: Gleitelement
- 30: Außenfläche
- 31: Gehäuseoberfläche
- 32: Absatz
- 33: Zwischenwand
- 34: Anschluss
- 35: Durchführung
- 36: Ansteckpunkt
- 37: Leiterplatte
- aa: Dauerstromkontakt
- bb: Automatikstromkontakt
- cc: Sensor

## Patentansprüche

1. Beatmungsgerät mit einem Gehäuse (1), wenigstens einer Atemgasquelle, wenigstens einem Steuersystem (3, 4 oder 5), wenigstens einer Schnittstelle (6) zur Verbindung mit einem Datenübertragungsstick (19) und/oder einem Datenspeicherstick (20), wobei das Gehäuse (1) eine Aufnahme (7) für die Schnittstelle (6) aufweist und die Aufnahme (7) mit der Schnittstelle (6) in einer Vertiefung (9) des Gehäuses (1) angeordnet ist, wobei die Vertiefung (9) als Schacht (10) ausgebildet ist und der Schacht eine Öffnung (11) aufweist und wobei sich von der Öffnung (11) in Richtung eines Innenraumes des Beatmungsgerätes zumindest zwei Umfangwände (12, 13, 14, 15) bis zu einem Boden (8) erstrecken und wobei die Aufnahme (7) mit der Schnittstelle (6) im Bereich des Bodens (8) oder einer Umfangswand (12, 13, 14, 15) der Vertiefung (9) angeordnet ist, **dadurch gekennzeichnet, dass** zumindest eine Umfangswand (12, 13, 14, 15) Führungsmittel aufweist, um einen Datenübertragungsstick (19) und/oder einen Datenspeicherstick (20) zu führen und im montierten Zustand zu stützen und die Vertiefung (9) durch einen Deckel (16) verschlossen ist und die Schnittstelle (6) einen Spritzwasser- und Berührungsschutz aufweist.

2. Beatmungsgerät nach zumindest einem der vorhergehenden Ansprüche, **dadurch gekennzeichnet, dass** die Vertiefung (9) durch den mit dem Gehäuse (1) im Wesentlichen flächenbündig abschließenden Deckel (16) verschlossen ist.

3. Beatmungsgerät nach zumindest einem der vorhergehenden Ansprüche, **dadurch gekennzeichnet, dass** der Deckel (16) relativ zum Gehäuse (1) bewegbar angeordnet ist und an dem Gehäuse befestigt ist und der Deckel (16) über zumindest eine Armatur (23) und/oder eine Oberflächenstrukturierung (24) verfügt, um ein Öffnen des Deckels (16) zu vereinfachen.

4. Beatmungsgerät nach zumindest einem der vorhergehenden Ansprüche, **dadurch gekennzeichnet, dass** im Gehäuse (1) und/oder der Umfangswand (12, 13, 14, 15) ein Mechanismus eingebaut ist, welcher bei Druckausübung den Deckel (16) zumindest teilweise öffnet und bei erneuter Druckausübung wieder in die Ausgangsposition zurückkehrt.

5. Beatmungsgerät nach zumindest einem der vorhergehenden Ansprüche **dadurch gekennzeichnet, dass** die Aufnahme (7) mit der Schnittstelle (6) sowie die Vertiefung (9) gegenüber dem Innenraum des Beatmungsgeräts abgedichtet ist.

6. Beatmungsgerät nach zumindest einem der vorhergehenden Ansprüche, **dadurch gekennzeichnet, dass** der Deckel (16) mit einem Werkzeug verschließbar ist.

7. Beatmungsgerät nach zumindest einem der vorhergehenden Ansprüche, **dadurch gekennzeichnet, dass** der Deckel (16) im geschlossenen Zustand in einer entsprechenden Vorrichtung in den Umfangswänden (12, 13, 14, 15) und/oder dem Gehäuserand (18) und/oder im/am Gehäuse (1) einrastet.

8. Beatmungsgerät nach zumindest einem der vorhergehenden Ansprüche, **dadurch gekennzeichnet, dass** die Vertiefung (9) in der Außenfläche (30) des Gehäuses (1) beabstandet zum Gehäuserand (18) angeordnet ist.

9. Beatmungsgerät nach zumindest einem der vorhergehenden Ansprüche, **dadurch gekennzeichnet, dass** die Schnittstelle (6) eine Kontaktierung mit einer Vielzahl unterschiedlicher Datenübertragungssticks (19) und/oder einer Vielzahl unterschiedlicher Datenspeichersticks (20) ermöglicht.

10. Beatmungsgerät nach zumindest einem der vorhergehenden Ansprüche, **dadurch gekennzeichnet, dass** die Aufnahme (7) mit der Schnittstelle (6) derart in dem Gehäuse versenkt angeordnet ist, dass ein Datenübertragungsstick (19) der in der Aufnahme und in der Schnittstelle (6) steckt, vollständig in der Vertiefung (9) angeordnet ist und maximal flächenbündig mit der Gehäuseoberfläche (31) des Gehäuses (1) abschließt nicht aber über die Gehäuseoberfläche (31) hinausragt und die Schnittstelle (6) und/oder die Aufnahme (7) und/oder die Vertiefung (9) über eine Entnahmevorrichtung mit Rückstellfunktion verfügt und der Datenübertragungsstick (19) oder Datenspeicherstick (20) durch die Betätigung der Entnahmevorrichtung in eine Position geführt wird, in der dieser nicht mehr flächenbündig mit dem Gehäuse (1) abschließt oder innerhalb des Gehäuses (1) angeordnet ist, sondern zumindest teilweise über das Gehäuse (1) hinausragt.

11. Beatmungsgerät nach zumindest einem der vorhergehenden Ansprüche, **dadurch gekennzeichnet, dass** die Vertiefung (9) über Eingriffsbereiche (22) für Finger und/oder Daumen für eine einfache Zug-Entnahme des Datenübertragungssticks (19) und/oder Datenspeichersticks (20) von der Schnittstelle (6) verfügt.

12. Beatmungsgerät nach zumindest einem der vorhergehenden Ansprüche, **dadurch gekennzeichnet, dass** die Schnittstelle (6) den Datenübertragungsstick (19) mit einer im Beatmungsgerät vorhandenen elektronischen Leiterplatte direkt oder über eine Kabelverbindung elektrisch und optional mechanisch verbindet und wobei die Schnittstelle (6) mit einer Steuereinheit des Beatmungsgerätes elektrisch leitend in Kontakt steht und zur Kommunikation von Datensignalen mit dem Beatmungsgerät ausgebildet ist.

13. Beatmungsgerät nach zumindest einem der vorhergehenden Ansprüche, **dadurch gekennzeichnet, dass** die Schnittstelle (6) einen Dauerstromkontakt und/oder einen Automatikstromkontakt aufweist, wobei dem Automatikstromkontakt ein Sensor zugeordnet ist, der zum Erfassen einer mechanischen Belegung der Schnittstelle ausgebildet ist.

14. Beatmungsgerät nach zumindest einem der vorhergehenden Ansprüche, **dadurch gekennzeichnet, dass** der Datenübertragungsstick (19) als ein in die Aufnahme (7) einführbares und in die Schnittstelle (6) steckbares Einführmodul ausgeführt ist, wobei die Schnittstelle (6) durch die Verbindung eines Datenübertragungsstick (19) eine Sende-/Empfangsanlage darstellet, zum Aufbau einer Drahtlosverbindung ausgebildet ist.

15. Beatmungsgerät nach zumindest einem der vorhergehenden Ansprüche, **dadurch gekennzeichnet, dass** die Schnittstelle (6) und/oder die Aufnahme (7) und/oder der Boden (8) und/oder die Vertiefung (9) und/oder der Schacht (10) und/oder zumindest eine Umfangswand (12, 13, 14, 15) Mittel aufweist und/oder ausgebildet ist, elektrische Felder sowie hochfrequente Wellen in zumindest einer Sperrrichtung zu verhindern oder abzuschwächen.

16. Beatmungsgerät nach zumindest einem der vorhergehenden Ansprüche **dadurch gekennzeichnet, dass** der Deckel (16) elektronisch verriegelt werden kann, wobei die elektronische Verriegelung des Deckels (16) durch eine Authentifizierung aufgehoben werden kann.

17. Beatmungsgerät nach zumindest einem der vorhergehenden Ansprüche, **dadurch gekennzeichnet, dass** in der Vertiefung (9) zusätzlich zu der Schnittstelle (6) zumindest ein weiteres Bauteil und/oder ein weiterer Anschluss (34) angeordnet ist, wobei der Bereich der Schnittstelle (6) in der Vertiefung (9) zumindest teilweise durch eine zumindest teilweise ausgebildete Zwischenwand (33) von den weiteren Bauteilen und/oder Anschlüssen (34) räumlich getrennt ist.

## Claims

1. A ventilator comprising a housing (1), at least one respiratory gas source, at least one control system (3, 4 or 5), at least one interface (6) for connection to a data transmission stick (19) and/or a data storage stick (20), wherein the housing (1) comprises a receptacle (7) for the interface (6) and the receptacle (7) is arranged with the interface (6) in a depression (9) of the housing (1), wherein the depression (9) is designed as a shaft (10) and the shaft comprises an opening (11) and wherein at least two perimeter walls (12, 13, 14, 15) extend to a base (8) from the opening (11) in the direction of an interior of the ventilator and wherein the receptacle (7) is arranged with the interface (6) in the region of the base (8) or a perimeter wall (12, 13, 14, 15) of the depression (9), **characterized in that** at least one perimeter wall (12, 13, 14, 15) comprises guide means for guiding a data transmission stick (19) and/or a data storage stick (20) and for supporting same in the mounted state and the depression (9) is closed by means of a cover (16) and the interface (6) comprises protection against spray water and contact.

2. The ventilator according to at least one of the preceding claims, **characterized in that** the depression (9) is closed by means of the cover (16), which is substantially flush with the housing (1).

3. The ventilator according to at least one of the preceding claims, **characterized in that** the cover (16) is arranged so as to be movable relative to the housing (1) and is fastened to the housing and the cover (16) has at least one fitting (23) and/or surface structuring (24) for facilitating opening of the cover (16).

4. The ventilator according to at least one of the preceding claims, **characterized in that** a mechanism that at least partially opens the cover (16) when pressure is exerted and that returns to the initial position when pressure is exerted again is integrated in the housing (1) and/or perimeter wall (12, 13, 14, 15).

5. The ventilator according to at least one of the preceding claims, **characterized in that** the receptacle (7) with the interface (6) as well as the depression (9) are sealed with respect to the interior of the ventilator.

6. The ventilator according to at least one of the preceding claims, **characterized in that** the cover (16) can be closed using a tool.

7. The ventilator according to at least one of the preceding claims, **characterized in that** the cover (16) latches in a corresponding device in the perimeter walls (12, 13, 14, 15) and/or the housing edge (18) and/or in/on the housing (1) when closed.

8. The ventilator according to at least one of the preceding claims, **characterized in that** the depression (9) is arranged in the outer surface (30) of the housing (1) at a distance from the housing edge (18).

9. The ventilator according to at least one of the preceding claims, **characterized in that** the interface (6) allows for contacting with a large number of different data transmission sticks (19) and/or a large number of different data storage sticks (20).

10. The ventilator according to at least one of the preceding claims, **characterized in that** the receptacle (7) with the interface (6) is recessed in the housing such that a data transmission stick (19) inserted in the receptacle and in the interface (6) is arranged completely in the depression (9) and is maximally flush with the housing surface (31) of the housing (1) but does not protrude beyond the housing surface (31) and the interface (6) and/or the receptacle (7) and/or the depression (9) has a removal device with a reset function and the data transmission stick (19) or data storage stick (20) is guided by means of actuation of the removal device into a position in which it is no longer flush with the housing (1) or arranged inside the housing (1), but rather protrudes beyond the housing (1) at least in part.

11. The ventilator according to at least one of the preceding claims, **characterized in that** the depression (9) has engaging regions (22) for fingers and/or thumbs for easily pulling the data transmission stick (19) and/or data storage stick (20) out of the interface (6).

12. The ventilator according to at least one of the preceding claims, **characterized in that** the interface (6) electrically and optionally mechanically connects the data transmission stick (19) to an electronic circuit board provided in the ventilator directly or via a cable connection and wherein the interface (6) is in electrically conductive contact with a control unit of the ventilator and is designed to communicate data signals with the ventilator.

13. The ventilator according to at least one of the preceding claims, **characterized in that** the interface (6) comprises a continuous current contact and/or an automatic current contact, wherein the automatic current contact is assigned a sensor that is designed to detect a mechanical assignment of the interface.

14. The ventilator according to at least one of the preceding claims, **characterized in that** the data transmission stick (19) is designed as an introductory module that can be introduced into the receptacle (7) and inserted in the interface (6), wherein the interface (6) constitutes a transceiver system that is designed to establish a wireless connection when a data transmission stick (19) is connected.

15. The ventilator according to at least one of the preceding claims, **characterized in that** the interface (6) and/or the receptacle (7) and/or the base (8) and/or the depression (9) and/or the shaft (10) and/or at least one perimeter wall (12, 13, 14, 15) comprises means and/or is designed for preventing or attenuating electrical fields and high-frequency waves in at least one reverse direction.

16. The ventilator according to at least one of the preceding claims, **characterized in that** the cover (16) can be electronically locked, wherein the electronic locking of the cover (16) can be canceled by means of authentication.

17. The ventilator according to at least one of the preceding claims, **characterized in that** at least one further component and/or one further connection (34) is arranged in the cavity (9) in addition to the interface (6), wherein the region of the interface (6) in the cavity (9) is spatially separated at least in part from the further components and/or connections (34) by an at least partially formed partition (33).

## Revendications

1. Appareil respiratoire avec un boîtier (1), au moins une source de gaz respiratoire, au moins un système de commande (3, 4 ou 5), au moins une interface (6) pour la connexion avec une clé de transmission de données (19) et/ou une clé de stockage de données (20), le boîtier (1) présentant un logement (7) pour l'interface (6) et le logement (7) étant disposé avec l'interface (6) dans une cavité (9) du boîtier (1), la cavité (9) étant conçue comme un puits (10) et le puits présentant une ouverture (11) et au moins deux parois périphériques (12, 13, 14, 15) s'étendant jusqu'à un fond (8) à partir de l'ouverture (11) dans la direction d'un espace intérieur de l'appareil respiratoire, et le logement (7) avec l'interface (6) étant disposé dans la région du fond (8) ou d'une paroi périphérique (12, 13, 14, 15) de la cavité (9), **caractérisé en ce qu'**au moins une paroi périphérique (12, 13, 14, 15) présente des moyens de guidage pour guider, et soutenir à l'état monté, une clé de transmission de données (19) et/ou une clé de stockage de données (20) et **en ce que** la cavité (9) est fermée par un couvercle (16) et **en ce que** l'interface (6) présente une protection contre les projections d'eau et contre les contacts.

2. Appareil respiratoire selon au moins l'une des revendications précédentes, **caractérisé en ce que** la cavité (9) est fermée par le couvercle (16) se trouvant essentiellement en affleurement avec le boîtier (1).

3. Appareil respiratoire selon au moins l'une des revendications précédentes, **caractérisé en ce que** le couvercle (16) est disposé de façon déplaçable relativement au boîtier (1) et est fixé au boîtier et **en ce que** le couvercle (16) dispose d'au moins une garniture (23) et/ou d'une structuration de surface (24) afin de faciliter une ouverture du couvercle (16).

4. Appareil respiratoire selon au moins l'une des revendications précédentes, **caractérisé en ce qu'**un mécanisme est intégré dans le boîtier (1) et/ou dans la paroi périphérique (12, 13, 14, 15), lequel ouvre au moins partiellement le couvercle (16) lors d'une application de pression et remet celui-ci dans la position initiale lors d'une nouvelle application de pression.

5. Appareil respiratoire selon au moins l'une des revendications précédentes, **caractérisé en ce que** le logement (7) avec l'interface (6) et la cavité (9) sont étanches vis-à-vis de l'espace intérieur de l'appareil respiratoire.

6. Appareil respiratoire selon au moins l'une des revendications précédentes, **caractérisé en ce que** le couvercle (16) est fermable avec un outil.

7. Appareil respiratoire selon au moins l'une des revendications précédentes, **caractérisé en ce que,** à l'état fermé, le couvercle (16) s'engage dans un dispositif correspondant dans les parois périphériques (12, 13, 14, 15) et/ou dans le bord du boîtier (18) et/ou dans le/sur le boîtier (1).

8. Appareil respiratoire selon au moins l'une des revendications précédentes, **caractérisé en ce que** la cavité (9) est disposée dans la surface extérieure (30) du boîtier (1) à distance du bord de boîtier (18).

9. Appareil respiratoire selon au moins l'une des revendications précédentes, **caractérisé en ce que** l'interface (6) permet l'établissement d'un contact avec une multiplicité de différentes clés de transmission de données (19) et/ou une multiplicité de différentes clés de stockage de données (20).

10. Appareil respiratoire selon au moins l'une des revendications précédentes, **caractérisé en ce que** le logement (7) avec l'interface (6) est disposé de façon encastrée dans le boîtier de telle sorte qu'une clé de transmission de données (19), laquelle est enfoncée dans le logement et dans l'interface (6), est disposée entièrement dans la cavité (9) et se trouve au maximum en affleurement avec la surface de boîtier (31) du boîtier (1), ne dépasse pas, toutefois, au-dessus de la surface de boîtier (31) et **en ce que** l'interface (6) et/ou le logement (7) et/ou la cavité (9) disposent d'un dispositif de retrait avec fonction de rappel et **en ce que** la clé de transmission de données (19) ou la clé de stockage de données (20) est guidée par l'actionnement du dispositif de retrait dans une position dans laquelle elle ne se trouve plus en affleurement avec le boîtier (1) ou n'est plus disposée à l'intérieur du boîtier (1), mais dépasse au moins partiellement au-dessus du boîtier (1).

11. Appareil respiratoire selon au moins l'une des revendications précédentes, **caractérisé en ce que** la cavité (9) dispose de régions de prise (22) pour les doigts et/ou pour les pouces, pour un retrait par traction simple de la clé de transmission de données (19) et/ou de la clé de stockage de données (20) hors de l'interface (6).

12. Appareil respiratoire selon au moins l'une des revendications précédentes, **caractérisé en ce que** l'interface (6) connecte électriquement et facultativement mécaniquement, directement ou par une connexion par câble, la clé de transmission de données (19) avec un circuit imprimé électronique présent dans l'appareil respiratoire, et l'interface (6) étant en contact de façon électriquement conductrice avec une unité de commande de l'appareil respiratoire et étant conçue pour la communication de signaux de données avec l'appareil respiratoire.

13. Appareil respiratoire selon au moins l'une des revendications précédentes, **caractérisé en ce que** l'interface (6) présente un contact de courant permanent et/ou un contact de courant automatique, un capteur étant attribué au contact de courant automatique, lequel est conçu pour la détection d'une occupation mécanique de l'interface.

14. Appareil respiratoire selon au moins l'une des revendications précédentes, **caractérisé en ce que** la clé de transmission de données (19) est réalisée comme un module d'insertion insérable dans le logement (7) et enfonçable dans l'interface (6), l'interface (6) représentant une installation d'émission/réception par la connexion d'une clé de transmission de données (19), étant conçue pour l'établissement d'une connexion sans fil.

15. Appareil respiratoire selon au moins l'une des revendications précédentes, **caractérisé en ce que** l'interface (6) et/ou le logement (7) et/ou le fond (8) et/ou la cavité (9) et/ou le puits (10) et/ou au moins une paroi périphérique (12, 13, 14, 15) présentent des moyens et/ou sont conçus pour empêcher ou atténuer des champs électriques ainsi que des ondes haute fréquence dans au moins un sens bloquant.

16. Appareil respiratoire selon au moins l'une des revendications précédentes **caractérisé en ce que** le couvercle (16) peut être verrouillé électroniquement, le verrouillage électronique du couvercle (16) pouvant être annulé par une authentification.

17. Appareil respiratoire selon au moins l'une des revendications précédentes, **caractérisé en ce qu'**au moins un autre composant et/ou un autre raccordement (34) sont disposés dans la cavité (9) en plus de l'interface (6), la région de l'interface (6) dans la cavité (9) étant séparée spatialement des autres composants et/ou raccordements (34) au moins partiellement par une paroi intermédiaire (33) au moins partiellement formée.
